(19) **Europäisches Patentamt / European Patent Office / Office européen des brevets**

(11) **EP 4 331 678 A1**

(12) **EUROPEAN PATENT APPLICATION**
published in accordance with Art. 153(4) EPC

(43) Date of publication:
06.03.2024 Bulletin 2024/10

(21) Application number: 22794899.9

(22) Date of filing: 26.04.2022

(51) International Patent Classification (IPC):
$A61P\ 35/00^{(2006.01)}$    $C07K\ 16/28^{(2006.01)}$
$A61K\ 35/17^{(2015.01)}$    $C12N\ 15/13^{(2006.01)}$
$C12N\ 5/10^{(2006.01)}$    $C07K\ 19/00^{(2006.01)}$

(52) Cooperative Patent Classification (CPC):
A61K 35/17; A61P 35/00; C07K 16/00;
C07K 16/28; C07K 19/00; C12N 5/10

(86) International application number:
PCT/CN2022/089287

(87) International publication number:
WO 2022/228429 (03.11.2022 Gazette 2022/44)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 27.04.2021 CN 202110462389

(71) Applicant: Oricell Therapeutics Co., Ltd.
Shanghai 201203 (CN)

(72) Inventors:
• CHEN, Siye
  Shanghai 201203 (CN)
• XU, Yanhong
  Shanghai 201203 (CN)
• GUO, Hao
  Shanghai 201203 (CN)

• YANG, Yue
  Shanghai 201203 (CN)
• LI, Huijiao
  Shanghai 201203 (CN)
• XU, Zhifeng
  Shanghai 201203 (CN)
• SUN, Yanan
  Shanghai 201203 (CN)
• YANG, Huanfeng
  Shanghai 201203 (CN)
• HE, Xiaowen
  Shanghai 201203 (CN)

(74) Representative: Weickmann & Weickmann
PartmbB
Postfach 860 820
81635 München (DE)

Remarks:
The complete document including Reference
Table(s) and the Sequence Listing(s) can be
downloaded from the EPO website

(54) **BCMA-TARGETING CHIMERIC ANTIGEN RECEPTOR AND USE THEREOF**

(57) Provided are a BCMA-targeting chimeric antigen receptor (CAR) and the use thereof. The CAR comprises a BCMA binding domain, and the BCMA binding domain comprises an antibody specifically binding to BCMA or an antigen-binding fragment thereof, wherein the antibody comprises a light-chain complementarity determining region 3 (HCDR3), and the HCDR3 comprises an amino acid sequence as shown in SEQ ID NO: 25. Further provided are an immune effector cell and a pharmaceutical composition comprising the CAR and a method for treating a disease or conditions associated with BCMA expression.

EP 4 331 678 A1

**Description**

FIELD OF THE INVENTION

This application relates to the field of biomedicine, and in particular to a chimeric antigen receptor targeting BCMA and a use thereof.

BACKGROUND OF THE INVENTION

BCMA (B cell maturation antigen) mainly regulates the proliferation and survival of B cells, as well as the mature differentiation of B cells into plasma cells (PCs). During the differentiation process to PCs, BCMA molecules are gradually induced to express. Since BCMA molecules are only expressed on the surface of PCs and plasmablast membranes and are not expressed on most B cells, hematopoietic stem cells, and other normal tissues, BCMA molecules have become one of the most ideal target molecules for treating MM.

Chimeric antigen receptor T (CAR-T) cell therapy is a precision targeted therapy that has emerged in recent years and is currently one of the hottest research directions in the field of tumor immunotherapy. The basic principle of this therapy is to transform T lymphocytes through genetic engineering technology into CAR-T cells with specific recognition capabilities, which are expanded and cultured in vitro and then reinfused into the patient's body to attack and kill specific tumor cells. CAR-T cell therapy can be applied to BCMA-targeted tumor immunotherapy.

However, there is no any CAR-T product targeting BCMA at present. In view of BCMA's effectiveness as a therapeutic target in B-cell malignancies, especially in multiple myeloma, there is an urgent need to develop new cell therapies in the field to achieve therapeutic goals by acting on BCMA.

SUMMARY OF THE INVENTION

This application provides a chimeric antigen receptor (CAR) targeting BCMA. The CAR of this application can be stably and efficiently expressed on the surface of immune effector cells (for example, T cells). The immune effector cells including the CAR have one or more of the following properties: (1) killing BCMA-positive target cells with high killing toxicity, (2) secreting a large amount of cytokines under stimulation of target cells, (3) high targeted proliferation ability, and (4) inhibiting tumor growth and eliminating tumors.

In one aspect, this application provides a chimeric antigen receptor (CAR), comprising a BCMA binding domain, and said BCMA binding domain comprises an antibody that specifically binds to BCMA or an antigen-binding fragment of the antibody, wherein said antibody comprises a light-chain complementarity determining region 3 (HCDR3), and said HCDR3 comprises an amino acid sequence as set forth in SEQ ID NO: 25.

In certain embodiments, said antibody that specifically binds to BCMA comprises a light-chain complementarity determining region 2 (HCDR2), and said HCDR2 comprises an amino acid sequence as set forth in SEQ ID NO: 41.

In certain embodiments, said HCDR2 of said antibody that specifically binds to BCMA comprises an amino acid sequence as set forth in any one of SEQ ID NOs: 22-24.

In certain embodiments, said antibody that specifically binds to BCMA comprises a light-chain complementarity determining region 1 (HCDR1), and said HCDR1 comprises an amino acid sequence as set forth in SEQ ID NO: 21.

In certain embodiments, said antibody that specifically binds to BCMA comprises an HCDR1, an HCDR2, and an HCDR3, wherein said HCDR1 comprises an amino acid sequence as set forth in SEQ ID NO: 21, said HCDR2 comprises an amino acid sequence as set forth in SEQ ID NO: 41, and said HCDR3 comprises an amino acid sequence as set forth in SEQ ID NO: 25.

In certain embodiments, said antibody that specifically binds to BCMA comprises an HCDR1, an HCDR2, and an HCDR3, wherein said HCDR1 comprises an amino acid sequence as set forth in SEQ ID NO: 21, said HCDR2 comprises an amino acid sequence as set forth in any one of SEQ ID NOs: 22-24, and said HCDR3 comprises an amino acid sequence as set forth in SEQ ID NO: 25.

In certain embodiments, said antibody that specifically binds to BCMA comprises any set of HCDR1, HCDR2, and HCDR3, selected from the groups consisting of : (1) HCDR1: SEQ ID NO: 21, HCDR2: SEQ ID NO: 22, and HCDR3: SEQ ID NO: 25; (2) HCDR1: SEQ ID NO: 21, HCDR2: SEQ ID NO: 23, and HCDR3: SEQ ID NO: 25; and (3) HCDR1: SEQ ID NO: 21, HCDR2: SEQ ID NO: 24, and HCDR3: SEQ ID NO: 25.

In certain embodiments, said antibody that specifically binds to BCMA comprises a light chain variable region (VH), said VH comprises a framework region H-FR1, a C terminal of said H-FR1 is directly or indirectly linked to an N terminal of said HCDR1, and said H-FR1 comprises an amino acid sequence as set forth in SEQ ID NO: 42.

In certain embodiments, said H-FR1 of said antibody that specifically binds to BCMA comprises an amino acid sequence as set forth in any one of SEQ ID NOs: 26-29.

In certain embodiments, said VH of said antibody that specifically binds to BCMA comprises a framework region H-FR2, and said H-FR2 is located between said HCDR1 and said HCDR2 and comprises an amino acid sequence as set forth in SEQ ID NO: 43.

In certain embodiments, said H-FR2 of said antibody that specifically binds to BCMA comprises an amino acid sequence as set forth in any one of SEQ ID NOs: 30-32.

In certain embodiments, said VH of said antibody that specifically binds to BCMA comprises a framework region H-FR3, and said H-FR3 is located between said HCDR2 and said HCDR3 and comprises an amino acid sequence as set forth in SEQ ID NO: 44.

In certain embodiments, said H-FR3 of said antibody that specifically binds to BCMA comprises an amino acid sequence as set forth in any one of SEQ ID NOs: 33-36.

In certain embodiments, said VH of said antibody that specifically binds to BCMA comprises a framework region H-FR4, an N terminal of said H-FR4 is linked to a C terminal of said HCDR3, and said H-FR4 comprises an amino acid sequence as set forth in SEQ ID NO: 37.

In certain embodiments, said antibody that specifically binds to BCMA comprises an H-FR1, an H-FR2, an H-FR3, and an H-FR4, wherein said H-FR1 comprises an amino acid sequence as set forth in SEQ ID NO: 42, said H-FR2 comprises an amino acid sequence as set forth in SEQ ID NO: 43, said H-FR3 comprises an amino acid sequence as set forth in SEQ ID NO: 44, and said H-FR4 comprises an amino acid sequence as set forth in SEQ ID NO: 37.

In certain embodiments, in said antibody that specifically binds to BCMA, said H-FR1 comprises an amino acid sequence as set forth in any one of SEQ ID NOs: 26-29, said H-FR2 comprises an amino acid sequence as set forth in any one of SEQ ID NOs: 30-32, said H-FR3 comprises an amino acid sequence as set forth in any one of SEQ ID NOs: 33-36, and said H-FR4 comprises an amino acid sequence as set forth in SEQ ID NO: 37.

In certain embodiments, said antibody that specifically binds to BCMA comprises any set of H-FR1, H-FR2, H-FR3, and H-FR4, selected from the groups consisting of : (1) H-FR1: SEQ ID NO: 26, H-FR2: SEQ ID NO: 30, H-FR3: SEQ ID NO: 33, and H-FR4: SEQ ID NO: 37; (2) H-FR1: SEQ ID NO: 27, H-FR2: SEQ ID NO: 31, H-FR3: SEQ ID NO: 34, and H-FR4: SEQ ID NO: 37; (3) H-FR1: SEQ ID NO: 28, H-FR2: SEQ ID NO: 32, H-FR3: SEQ ID NO: 35, and H-FR4: SEQ ID NO: 37; and (4) H-FR1: SEQ ID NO: 29, H-FR2: SEQ ID NO: 30, H-FR3: SEQ ID NO: 36, and H-FR4: SEQ ID NO: 37.

In certain embodiments, said antibody that specifically binds to BCMA comprises a light chain variable region (VH), and said VH comprises an amino acid sequence as set forth in SEQ ID NO: 46.

In certain embodiments, said VH of said antibody that specifically binds to BCMA comprises an amino acid sequence as set forth in any one of SEQ ID NOs: 5-9.

In certain embodiments, said antibody that specifically binds to BCMA comprises a light-chain complementarity determining region 3 (LCDR3), and said LCDR3 comprises an amino acid sequence as set forth in SEQ ID NO: 40.

In certain embodiments, said LCDR3 of said antibody that specifically binds to BCMA comprises an amino acid sequence as set forth in any one of SEQ ID NOs: 15-16.

In certain embodiments, said antibody that specifically binds to BCMA comprises a light-chain complementarity determining region 2 (LCDR2), and said LCDR2 comprises an amino acid sequence as set forth in SEQ ID NO: 39.

In certain embodiments, said LCDR2 of said antibody that specifically binds to BCMA comprises an amino acid sequence as set forth in any one of SEQ ID NOs: 13-14.

In certain embodiments, said antibody that specifically binds to BCMA comprises a light-chain complementarity determining region 1 (LCDR1), and said LCDR1 comprises an amino acid sequence as set forth in SEQ ID NO: 38.

In certain embodiments, said LCDR1 of said antibody that specifically binds to BCMA comprises an amino acid sequence as set forth in any one of SEQ ID NOs: 10-12.

In certain embodiments, said antibody that specifically binds to BCMA comprises an LCDR1, an LCDR2, and an LCDR3, wherein said LCDR1 comprises an amino acid sequence as set forth in SEQ ID NO: 38, said LCDR2 comprises an amino acid sequence as set forth in SEQ ID NO: 39, and said LCDR3 comprises an amino acid sequence as set forth in SEQ ID NO: 40.

In certain embodiments, said antibody that specifically binds to BCMA comprises an LCDR1, an LCDR2, and an LCDR3, wherein said LCDR1 comprises an amino acid sequence as set forth in any one of SEQ ID NOs: 10-12, said LCDR2 comprises an amino acid sequence as set forth in any one of SEQ ID NO: 13-14, and said LCDR3 comprises an amino acid sequence as set forth in any one of SEQ ID NO: 15-16.

In certain embodiments, said antibody specifically binding to BCMA comprises LCDR1, LCDR2, and LCDR3, and said that specifically binds to BCMA comprises any set of LCDR1, LCDR2, and LCDR3 comprises any set of amino acid sequence, selected from the groups consisting of : (1) LCDR1: SEQ ID NO: 10, LCDR2: SEQ ID NO: 13, and LCDR3: SEQ ID NO: 15; (2) LCDR1: SEQ ID NO: 11, LCDR2: SEQ ID NO: 13, and LCDR3: SEQ ID NO: 16; (3) LCDR1: SEQ ID NO: 12, LCDR2: SEQ ID NO: 13, and LCDR3: SEQ ID NO: 16; and (4) LCDR1: SEQ ID NO: 12, LCDR2: SEQ ID NO: 14, and LCDR3: SEQ ID NO: 16.

In certain embodiments, said antibody that specifically binds to BCMA comprises a light chain variable region (VL), said VL comprises a framework region L-FR1, a C terminal of said L-FR1 is directly or indirectly linked to an N terminal of said LCDR1, and said L-FR1 comprises an amino acid sequence as set forth in SEQ ID NO: 17.

In certain embodiments, said VL of said antibody that specifically binds to BCMA comprises a framework region L-FR2, and said L-FR2 is located between said LCDR1 and said LCDR2 and comprises an amino acid sequence as set forth in SEQ ID NO: 18.

In certain embodiments, said VL of said antibody that specifically binds to BCMA comprises a framework region L-FR3, and said L-FR3 is located between said LCDR2 and said LCDR3 and comprises an amino acid sequence as set forth in SEQ ID NO: 19.

In certain embodiments, said VL of said antibody that specifically binds to BCMA comprises a framework region L-FR4, an N terminal of said L-FR4 is linked to a C terminal of said LCDR3, and said L-FR4 comprises an amino acid sequence as set forth in SEQ ID NO: 20.

In certain embodiments, said antibody that specifically binds to BCMA comprises an L-FR1, an L-FR2, an L-FR3, and an L-FR4, wherein said L-FR1 comprises an amino acid sequence as set forth in SEQ ID NO: 17, said L-FR2 comprises an amino acid sequence as set forth in SEQ ID NO: 18, said L-FR3 comprises an amino acid sequence as set forth in SEQ ID NO: 19, and said L-FR4 comprises an amino acid sequence as set forth in SEQ ID NO: 20.

In certain embodiments, said antibody that specifically binds to BCMA comprises a light chain variable region (VL), and said VL comprises an amino acid sequence as set forth in SEQ ID NO: 45.

In certain embodiments, said VL of said antibody that specifically binds to BCMA comprises an amino acid sequence as set forth in any one of SEQ ID NOs: 1-4.

In certain embodiments, said antibody that specifically binds to BCMA comprises a VH and a VL, said VH comprises an amino acid sequence as set forth in SEQ ID NO: 46, and said VL comprises an amino acid sequence as set forth in SEQ ID NO: 45.

In certain embodiments, said antibody that specifically binds to BCMA comprises a VH and a VL, said VH comprises an amino acid sequence as set forth in any one of SEQ ID NOs: 5-9, and said VL comprises an amino acid sequence as set forth in any one of SEQ ID NOs: 1-4.

In certain embodiments, said antibody that specifically binds to BCMA comprises VH and VL, and said VH and VL comprises any set of amino acid sequence, selected from the groups consisting of: (1) VH: SEQ ID NO: 5 and VL: SEQ ID NO: 1; (2) VH: SEQ ID NO: 6 and VL: SEQ ID NO: 2; (3) VH: SEQ ID NO: 7 and VL: SEQ ID NO: 2; (4) VH: SEQ ID NO: 8 and VL: SEQ ID NO: 2; (5) VH: SEQ ID NO: 9 and VL: SEQ ID NO: 3; (6) VH: SEQ ID NO: 8 and VL: SEQ ID NO: 3; (7) VH: SEQ ID NO: 9 and VL: SEQ ID NO: 4; and (8) VH: SEQ ID NO: 10 and VL: SEQ ID NO: 4.

In certain embodiments, said BCMA binding domain comprises a scFv, and said scFv comprises said VH and said VL of said antibody that specifically binds to BCMA.

In certain embodiments, in said scFv, the C terminal of said VH and the N terminal of said VL are directly or indirectly linked.

In certain embodiments, in said scFv, the C terminal of said VL and the N terminal of said VL are directly or indirectly linked.

In certain embodiments, said scFv comprises a linker peptide between said VH and said VL, and said linker peptide comprises an amino acid sequence as set forth in SEQ ID NO: 58.

In certain embodiments, said scFv comprises an amino acid sequence as set forth in any one of SEQ ID NOs: 49-56.

In certain embodiments, the CAR comprises a transmembrane domain, and said transmembrane domain comprises transmembrane domains derived from proteins selected from the group consisting of: CD28, CD3e, CD45, CD4, CD5, CD8, CD9, CD16, CD22, CD33, CD37, CD64, CD80, CD86, CD134, CD137, and CD154. In certain embodiments, said transmembrane domain is derived from CD8, and said transmembrane domain comprises an amino acid sequence as set forth in SEQ ID NO: 61.

In certain embodiments, the CAR comprises a costimulatory domain, and said costimulatory domain comprises costimulatory domains derived from proteins selected from the group consisting of: CD137, CD28, 4-1BB, OX-40, and ICOS. In certain embodiments, said costimulatory domain is derived from CD137, and said costimulatory domain comprises an amino acid sequence as set forth in SEQ ID NO: 62.

In certain embodiments, the CAR comprises an intracellular signaling domain, and said intracellular signaling domain comprises an intracellular signaling domain derived from CD3ζ. In certain embodiments, said intracellular signaling domain comprises an amino acid sequence as set forth in SEQ ID NO: 63.

In certain embodiments, the CAR comprises a hinge region, and said hinge region comprises a hinge region derived from CD8. In certain embodiments, said hinge region is derived from CD8 and comprises an amino acid sequence as set forth in SEQ ID NO: 60.

In certain embodiments, the CAR comprises a signal peptide. In certain embodiments, said signal peptide comprises an amino acid sequence as set forth in SEQ ID NO: 59.

In certain embodiments, the CAR comprises L6-Li and said L6-Li is located at a C terminal of the intracellular signaling domain. In certain embodiments, said L6-Li comprises an amino acid sequence as set forth in SEQ ID NO: 107.

In certain embodiments, the CAR comprises an amino acid sequence as set forth in any one of SEQ ID NOs: 64-71 and 74. In certain embodiments, the CAR comprises a nucleotide sequence as set forth in any one of SEQ ID NOs: 95-102 and 105.

In another aspect, this application provides an isolated nucleic acid molecule, said nucleic acid molecule comprising a nucleotide sequence encoding the CAR of this application.

In certain embodiments, said isolated nucleic acid molecule further comprises a promoter located at a 5' terminal of the nucleotide sequence encoding said CAR. In certain embodiments, said promoter is a constitutive promoter. In certain embodiments, said promoter is an EF1α promoter.

In another aspect, this application provides a nucleic acid molecule encoding a CAR, said nucleic acid molecule encoding a CAR comprising a nucleotide sequence as set forth in any one of SEQ ID NOs: 95-102 and 105.

In another aspect, this application provides a vector comprising the nucleic acid molecule of this application.

In certain embodiments, the vector is selected from the group consisting of plasmids, retroviral vectors, and lentivirus vectors.

In another aspect, this application provides an immune effector cell comprising the CAR, the nucleic acid molecule, and/or the vector of this application. In certain embodiments, the immune effector cell is selected from T lymphocytes.

In another aspect, this application provides a method for preparing an immune effector cell, comprising: introducing the vector of this application into said immune effector cell.

In another aspect, this application provides a pharmaceutical composition comprising the CAR, the nucleic acid molecule, the vector, and/or the immune effector cell, and a pharmaceutically acceptable carrier.

In another aspect, this application further provides a use of the CAR, the nucleic acid molecule, the vector, the immune effector cell, and/or the pharmaceutical composition in the manufacture of a medicament, wherein said medicament is used for treating a disease and/or disorder associated with BCMA expression.

In certain embodiments, the disease or disorder associated with BCMA expression is a cancer or a malignant tumor. In certain embodiments, the disease or disorder associated with BCMA expression is a BCMA-positive tumor. In certain embodiments, the disease or disorder associated with BCMA expression is a solid tumor and/or a non-solid tumor. In certain embodiments, the disease or disorder associated with BCMA expression is myeloma. In certain embodiments, the disease or disorder associated with BCMA expression is multiple myeloma.

In another aspect, this application further provides a method for treating a disease or disorder associated with BCMA expression, comprising: administering the CAR, the nucleic acid molecule, the vector, the immune effector cell, and/or the pharmaceutical composition to a subject in need.

In certain embodiments, the disease or disorder associated with BCMA expression is a cancer or a malignant tumor. In certain embodiments, the disease or disorder associated with BCMA expression is a BCMA-positive tumor. In certain embodiments, the disease or disorder associated with BCMA expression is a solid tumor and/or a non-solid tumor. In certain embodiments, the disease or disorder associated with BCMA expression is myeloma. In certain embodiments, the disease or disorder associated with BCMA expression is multiple myeloma.

In another aspect, this application further provides the CAR, the nucleic acid molecule, the vector, the immune effector cell, and/or the pharmaceutical composition, used for treating a disease and/or disorder associated with BCMA expression.

In certain embodiments, the disease or disorder associated with BCMA expression is a cancer or a malignant tumor. In certain embodiments, the disease or disorder associated with BCMA expression is a BCMA-positive tumor. In certain embodiments, the disease or disorder associated with BCMA expression is a solid tumor and/or a non-solid tumor. In certain embodiments, the disease or disorder associated with BCMA expression is myeloma. In certain embodiments, the disease or disorder associated with BCMA expression is multiple myeloma.

Those skilled in the art can easily perceive other aspects and advantages of this application from the following detailed description. Only exemplary embodiments of this application are shown and described in detail below. As those skilled in the art will appreciate, from the content of the present application, those skilled in the art may make changes to the disclosed specific embodiments without departing from the spirit and scope of the invention to which the present application relates. Accordingly, the descriptions in the drawings and specification of this application are only exemplary and not restrictive.

BRIEF DESCRIPTION OF THE DRAWINGS

The specific features of the invention referred to this application are shown in the appended claims. The features and advantages of the invention to which this application relates can be better understood with reference to the exemplary embodiments and the accompanying drawings described in detail below. The drawings are described briefly as follows.

FIG. 1 shows an embodiment of the composition of CAR elements in CAR lentivirus vectors and their connection sequences.

FIG. 2 shows the expansion fold of each group of BCMA CAR-T cell comprising the CAR of this application during the culture process.

FIG. 3 shows the proportion of CAR-positive cells in each group of BCMA CAR-T cell comprising the CAR of this application.

FIG. 4 shows the killing toxicity of each group of BCMA CAR-T cell comprising the CAR of this application against the target cell, where A shows the results of MM1s cell, and B shows the results of K562 cell.

FIG. 5 shows the IFN-γ cytokine secretion after co-incubation of each group of BCMA CAR-T cell comprising the CAR of this application with the MM1s cell.

FIG. 6 shows the targeted expansion ability of each group of BCMA CAR-T cell comprising the CAR of this application for the MM1s cell.

FIG. 7 shows the killing toxicity of BCMA CAR-T cell based on a L6-Li CAR structure against the MM1s cell.

FIG. 8 shows the IFN-γ cytokine secretion after co-incubation of each group of BCMA CAR-T cell based on the L6-Li CAR structure with the MM1s cell.

FIG. 9 shows the ability of BCMA CAR-T cells based on the L6-Li CAR structure to inhibit and decrease tumors in tumor-bearing mice, where A shows the results of the experimental group and B shows the results of the control group.

FIG. 10 shows the metabolism of BCMA CAR-T cells based on the L6-Li CAR structure to inhibit and decrease tumors in tumor-bearing mice, where A shows the results of the experimental group and B shows the results of the control group.

FIG. 11 shows IFN-γ cytokine secretion caused by BCMA CAR-T cells based on the L6-Li CAR structure in tumor-bearing mice, where A shows the results of the experimental group and B shows the results of the control group.

FIG. 12 shows IL-2 cytokine secretion caused by BCMA CAR-T cells based on the L6-Li CAR structure in tumor-bearing mice.

DETAILED DESCRIPTION OF THE INVENTION

The implementation of the invention of this application will be described below with specific embodiments. Those skilled in the art can easily understand other advantages and effects of the invention of this application from the contents disclosed herein.

Definition of Terms

As used herein, the term "BCMA" is used interchangeably with "CD269", "BCM" and "TNFRSF17" and generally refers to B cell maturation antigen. The BCMA protein is a member of the tumor necrosis receptor family. As used herein, the term "BCMA" may include proteins containing mutations, for example, may include proteins containing point mutations, fragments, insertions, deletions and splice variants of full-length wild-type BCMA. The BCMA may include those from any vertebrate, for example, primates (e.g., humans or monkeys), rodents (mouse or rat), avian and/or livestock, and the like. As used herein, the term "BCMA" may also include a portion of the intact BCMA protein, as long as the relevant biological activity is retained. In this application, the BCMA may be human BCMA (GenBank ID: BAB60895.1). For example, human BCMA is typically a 184 amino acid long protein encoded by a 994 nucleotide long primary mRNA transcript (NM_001192.2).

As used herein, the term "binding domain" typically refers to a domain that (specifically) binds to a given target epitope or a given target site on a target molecule (antigen), or that interacts with, or recognizes a given target epitope or a given target site.

As used herein, the term "BCMA binding domain" typically refers to a domain that can specifically bind to a BCMA protein. For example, the BCMA binding domain may comprise a chimeric antigen receptor or a fragment thereof, an anti-BCMA antibody or an antigen-binding fragment thereof that specifically binds to a BCMA polypeptide expressed on a B cell. The terms "binding domain", "extracellular domain", "extracellular binding domain", "antigen-specific binding domain" and "extracellular antigen-specific binding domain" are used interchangeably in this application and may comprise a domain or fragment of a CAR that has the ability to specifically bind to a target antigen of interest (e.g., BCMA). The BCMA binding domain can be of natural, synthetic, semi-synthetic or recombinant origin.

As used herein, the term "antibody" typically refers to an immunoglobulin or a fragment or derivative thereof and encompasses any polypeptide comprising an antigen-binding site, whether produced in vitro or in vivo. The term includes, but is not limited to, polyclonal, monoclonal, monospecific, multispecific, non-specific, humanized, single-stranded, chimeric, synthetic, recombinant, hybrid, mutated and transplanted antibodies. Unless otherwise modified by the term "intact", as in "intact antibody", the term "antibody" also includes antibody fragments, such as Fab, F (ab') $_2$, Fv, scFv, Fd, dAb and other antibody fragments that maintain an antigen binding functionality, of, for example, specifically binding to BCMA. Typically, an antibody may comprise an immunoglobulin consisting of at least two heavy (H) chains and two light (L) chains interconnected by disulfide bonds, and comprise any molecule that contains an antigen-binding portion thereof. Each heavy chain consists of a heavy chain variable region (VH) and a heavy chain constant region. Each light chain consists of a light chain variable region (VL) and a light chain constant region. The VH and VL can be further distinguished into hypervariable regions called complementarity determining regions (CDRs), which are interspersed with more conserved regions called framework regions (FRs). Each VH and VL may be composed of three CDRs and four FR regions, which may be arranged in the following order from the amino terminal to the carboxyl terminal: FR1, CDR1, FR2, CDR2, FR3, CDR3 and FR4. The variable regions of the heavy and light chains contain binding domains that interact with an antigen.

As used herein, the term "scFv" typically refers to a single-chain antibody, which is an antibody composed of a heavy chain variable region and a light chain variable region that are linked directly or through a linking molecule (e.g., a linker peptide). The structure of the scFv, from the N terminal to the C terminal, may be heavy chain variable region-light chain variable region, light chain variable region-heavy chain variable region, heavy chain variable region-linker peptide-light chain variable region, or light chain variable region-linker peptide-heavy chain variable region.

As used herein, the term "chimeric antigen receptor (CAR)" typically refers to a fusion protein comprising an extracellular domain able to bind to an antigen and at least one intracellular domain. The CAR may be an antigen receptor formed by fusion of an antigen-binding region of an antibody that recognizes a tumor-associated antigen (TAA) and an intracellular signaling domain "immunoreceptor tyrosine-based activation motif (ITAM)". The basic structure of a CAR includes a tumor-associated antigen (TAA) binding region (e.g., scFv), an extracellular hinge region, a transmembrane region, and an intracellular signaling domain. In this application, the CAR may be combined with an immune effector cell receptor activation intracellular domain based on the antigen (e.g., BCMA) specificity of the antibody. Immune effector cells genetically modified to express CAR can specifically recognize and eliminate malignant cells expressing target antigens.

As used herein, the term "signaling domain" generally refers to a domain located inside a cell that is capable of transducing a signal. The intracellular signaling domain described herein can transmit signals into cells. Typically, a signaling domain is any piece of contiguous amino acid sequence that directs a protein to find a target. For example, in certain embodiments, the intracellular signaling domain can be selected from the group consisting of CD3$\zeta$ intracellular domain, CD28 intracellular domain, CD28 intracellular domain, 4-1BB intracellular domain, and OX40 intracellular domain.

As used herein, the term "costimulation" generally refers to the source of lymphocyte activation secondary signals, usually produced by the interaction of costimulatory molecules and their receptors on the surface of immune cells (T cell/ B cell or antigen presenting cell/T cell) involved in adaptive immunity. For example, complete activation of T cells depends on the action of dual signals and cytokines. The first signal of T cell activation comes from the specific binding of its receptor TCR to the antigen, that is, the T cell recognizes the antigen. The second signal of T cell activation comes from the costimulatory molecule, i.e., the interaction of a costimulatory molecule on APC with a corresponding receptor on the surface of the T cell.

As used herein, the term "costimulatory domain" typically refers to any amino acid sequence capable of interacting with a costimulatory molecule to produce costimulation.

As used herein, the term "hinge region" generally refers to a region in the CAR structure located between the antibody or antigen-binding fragment thereof (e.g., scFv) that specifically binds to BCMA and the transmembrane domain. The hinge region is typically derived from the IgG family, such as IgG1 and IgG4, and some are derived from IgD and CD8. Typically, the hinge region has a certain degree of flexibility, which affects the spatial constraint between the CAR molecule and its specific target, thereby affecting the contact between a CART cell and a tumor cell.

As used herein, the term "transmembrane domain" typically refers to a sequence in a cell surface protein that spans the cell membrane and may comprise a hydrophobic alpha helix. The transmembrane domain can be linked to the intracellular signaling domain to play a role in transmitting signals. The transmembrane domain typically contains three different structural regions: an N-terminal extracellular region, a middle conserved transmembrane stretching region, and a C-terminal cytoplasmic region. The transmembrane domain may also contain an intracellular or cytoplasmic region. The transmembrane domain described herein may be derived from any type I, type II or type III transmembrane protein.

As used herein, the term "signal peptide" typically refers to a leader sequence at the amino terminal (N-terminal) of the nascent CAR protein, which directs the nascent protein to the endoplasmic reticulum and subsequent surface expression during or after translation. The signal peptide is typically cleaved during this process. The signal peptide may be heterologous or homologous to the organism used to produce a polypeptide.

As used herein, the term "L6-Li" typically refers to the fusion protein of low-density lipoprotein receptor-related protein and leptin. For relevant information about L6-Li, please refer to Patent Application Publication No. WO2021057932A1. In this application, said L6-Li may comprise an amino acid sequence as set forth in SEQ ID NO: 107. In this application, the nucleic acid molecule encoding said L6-Li may comprise a nucleotide sequence as set forth in SEQ ID NO: 106.

As used herein, the term "promoter" generally refers to a deoxyribonucleic acid (DNA) sequence that enables the transcription of a specific gene. The promoter is recognized by RNA polymerase and begins transcription to synthesize RNA. In ribonucleic acid (RNA) synthesis, the promoter can interact with transcription factors that regulate gene transcription, controlling the initiation time and degree of gene expression (transcription). The promoter includes a core promoter region and a regulatory region, which are located in the regulatory sequence that controls gene expression and at upstream of the gene transcription start site (5' direction of the DNA antisense strand). They have no compilation function themselves. According to their mode of action and function, they are divided into three categories: constitutive promoters (that remain continuously active in most or all tissues), specific (tissue specific or developmental period specific) promoters and inducible promoters (regulated by external chemical or physical signals).

As used herein, the term "specifically binding" generally refers to a measurable and reproducible interaction, such as the binding between a target and an antibody, that determines the presence of the target in the presence of a heterogeneous population of molecules, including biomolecules. For example, an antibody specifically binding to a target (or an epitope) may be an antibody that binds to that target with greater affinity, avidity, easier, and/or for a longer duration than it binds to other targets. In certain embodiments, the antibody specifically binds to an epitope on a protein that is conservative among proteins of different species. In another embodiment, the "specifically binding" includes, but does not require exclusive binding.

As used herein, the term "nucleic acid molecule" typically refers to an isolated form of nucleotides, deoxyribonucleotides or ribonucleotides of any length or their analogs. In certain embodiments, the nucleic acid molecule described herein may be isolated from the natural environment. In certain embodiments, the nucleic acid molecule described herein may be produced or synthesized by: (i) vitro amplification, for example, polymerase chain reaction (PCR) amplification; (ii) clonal recombination; (iii) purification, for example, enzymatic digestion and gel electrophoresis fractionation, or (iv) synthesis, for example, chemical synthesis. In certain embodiments, the isolated nucleic acid is a nucleic acid molecule prepared by recombinant DNA technology. In this application, nucleic acids encoding the antibodies or antigen-binding fragments thereof may be prepared by a variety of methods known in the art, including, but not limited to, overlap extension PCR using restriction fragment manipulation or synthetic oligonucleotides. For specific operations, please refer to Sambrook et al., Molecular Cloning, A Laboratory Manual, Cold Spring Harbor Laboratory Press, Cold Spring Harbor, N.Y., 1989; and Ausube et al., Current Protocols in Molecular Biology, Greene Publishing and Wiley-Interscience, New York N.Y, 1993.

As used herein, the terms "polypeptide" and "protein" are used interchangeably and generally refer to a polymer of amino acid residues. The term also applies to amino acid polymers in which one or more amino acid residues are analogs or mimetics of the corresponding naturally occurring amino acids, as well as naturally occurring amino acid polymers and homologues. The term may also include amino acid polymers modified, for example, by the addition of sugar residues to form glycoproteins or by phosphorylation. Polypeptides and proteins may be produced from naturally occurring and non-recombinant cells or from genetically engineered or recombinant cells, and may comprise molecules having the amino acid sequence of the native protein, or molecules having deletions, additions and/or substitutions of one or more amino acids of the native sequence. The terms "polypeptide" and "protein" particularly include sequences in which one or more amino acids of the antigen-binding proteins described herein are deleted, added and/or substituted.

When referring to the amino acid sequence of a protein or the nucleotide sequence of a nucleic acid molecule, the present application also includes homologs of these sequences. As used herein, the term "homologue" typically refers to an amino acid sequence or a nucleotide sequence that has certain homology to a wild-type amino acid sequence and a wild-type nucleotide sequence. The term "homology" may be equivalent to sequence "identity". Homologous sequences may include amino acid sequences that may be at least 80% identical to the subject sequence 80%, 85%, 90%, 99.1%, 99.2%, 99.3%, 99.4%, 99.5%, 99.6%, 99.7%, 99.8%, or 99.9%. Typically, homologs will contain active sites, etc. that are identical to the subject amino acid sequence. Homology may be considered in terms of similarity (i.e., amino acid residues with similar chemical properties/functions), or homology may be expressed in terms of sequence identity. In this application, reference to a sequence having a percent identity over any one of the SEQ ID NOs of an amino acid sequence or a nucleotide sequence means a sequence having said percent identity over the entire length of the mentioned SEQ ID NO.

**[0091]** As used herein, the term "vector" refers to a nucleic acid delivery vehicle into which a polynucleotide encoding a protein can be inserted such that the protein can be expressed. The vector can be expressed by transforming, transducing or transfecting the host cell, so that the genetic material elements carried thereon can be expressed in the host cell. For example, the vector includes: plasmids; phagemids; cosmids; artificial chromosomes such as yeast artificial chromosomes (YACs), bacterial artificial chromosomes (BACs) or P1-derived artificial chromosomes (PACs); phages such as λ phage or M13 phage and animal viruses, etc. Animal virus species used as vectors include retroviruses (including lentiviruses), adenoviruses, adeno-associated viruses, herpesviruses (such as herpes simplex virus), poxviruses, baculoviruses, papillomaviruses, and papovavirus (e.g., SV40). A vector may comprise a variety of elements that control expression, including promoter sequence, transcriptional initiation sequence, enhancer sequence, selective element and reporter gene. In addition, the vector may also comprise replication initiation sites. The vector may also comprise components that assist its entry into cells, such as, but not exclusively, viral particles, liposomes, or protein coats.

As used herein, the term "immune effector cell" typically refers to cells involved in immune responses, for example, promoting immune effector responses. Examples of immune effector cells include T cells, e.g., $\alpha/\beta$ T cells and $\gamma/\delta$ T T cells, B cells, natural killer (NK) cells, natural killer T (NKT) cells, mast cells, and bone marrow-derived phagocytes.

As used herein, the term "pharmaceutical composition" typically refers to a composition suitable for administration to patients, human patients. For example, the pharmaceutical composition of this application may include the immune effector cell, CAR, nucleic acid molecule or vector of this application, and optionally a pharmaceutically acceptable carrier. The pharmaceutical composition comprises a potent biologically active form of the active ingredient and does not comprise additional ingredients that would be unacceptably toxic to the subject to whom the formulation is to be administered.

As used herein, the term "pharmaceutically acceptable carrier" generally refers to one or more non-toxic materials that do not interfere with the effectiveness of the biological activity of the active ingredient. Such formulations may conventionally comprise salts, buffers, preservatives, compatible carriers, and optionally other therapeutic agents.

The term "disease or disorder associated with BCMA expression" includes, but is not limited to, diseases associated with BCMA expression, or disorders associated with BCMA-expressing cells, including tumor cells of various cancers. An individual with a disease or disorder associated with BCMA expression has abnormal BCMA expression (e.g., BCMA overexpression), or a cell has abnormal BCMA expression (e.g., BCMA overexpression) compared to a normal organism or a normal cell.

As used herein, the term "cancer or malignant tumor" typically refers to a neoplasm or solid lesion formed by abnormal cell growth. In this application, the tumor may be a solid tumor or a non-solid tumor (e.g., a hematological tumor).

As used herein, the term "BCMA-positive tumor" typically refers to tumors in which BCMA protein is overexpressed compared to normal cells. For example, the BCMA-positive tumor may include non-solid tumors. For example, the BCMA-positive tumor may include myeloma. For example, the BCMA-positive tumor may include multiple myeloma.

As used herein, the term "comprising" typically means including specifically specified features, but not excluding other elements.

As used herein, the term "about" generally refers to a variation within a range of 0.5%-10% above or below a specified value, for example, a variation within a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5% or 10% above or below a specified value.

Detailed description

In one aspect, this application provides a chimeric antigen receptor (CAR), comprising a BCMA binding domain, and said BCMA binding domain comprises an antibody that specifically binds to BCMA or an antigen-binding fragment of the antibody.

BCMA binding domain

In this application, said antibody that specifically binds to BCMA or the antigen-binding fragment thereof in the CAR may comprise a heavy-chain complementarity determining region 3 (HCDR3), and said HCDR3 may comprise an amino acid sequence as set forth in SEQ ID NO: 25: DIRYVMDY (SEQ ID NO: 25). For example, said sequence can be divided according to Kabat.

In this application, said antibody or the antigen-binding fragment thereof may comprise a heavy-chain complementarity determining region 2 (HCDR2), and said HCDR2 may comprise an amino acid sequence as set forth in SEQ ID NO: 41: WINTETREPX$_{10}$YAYDFRX$_{17}$ (SEQ ID NO: 41), wherein X$_{10}$=A or T, X$_{17}$=G or S. For example, said sequence can be divided according to Kabat.

In this application, said HCDR2 may comprise an amino acid sequence as set forth in any one of SEQ ID NOs: 22-24.

In this application, said antibody or the antigen-binding fragment thereof may comprise a heavy-chain complementarity determining region 1 (HCDR1), and said HCDR1 may comprise an amino acid sequence as set forth in SEQ ID NO: 21: DYTIN (SEQ ID NO: 21). For example, said sequence can be divided according to Kabat.

In this application, said antibody or the antigen-binding fragment thereof may comprise an HCDR1, an HCDR2, and an HCDR3, and the amino acid sequences of said HCDR1, HCDR2 and HCDR3 may be as set forth in SEQ ID NO: 21, SEQ ID NO: 41, and SEQ ID NO: 25, respectively.

In this application, said antibody or the antigen-binding fragment thereof may comprise an HCDR1, an HCDR2, and an HCDR3, wherein said HCDR1 may comprise an amino acid sequence as set forth in SEQ ID NO: 21, said HCDR2 may comprise an amino acid sequence as set forth in any one of SEQ ID NOs: 22-24, and said HCDR3 may comprise an amino acid sequence as set forth in SEQ ID NO: 25.

In this application, said antibody or the antigen-binding fragment thereof may comprise an HCDR1, an HCDR2, and an HCDR3, wherein the amino acid sequences of said HCDR1, HCDR2, and HCDR3 may be as set forth in SEQ ID NO: 21, SEQ ID NO: 22, and SEQ ID NO: 25, respectively.

In this application, said antibody or the antigen-binding fragment thereof may comprise an HCDR1, an HCDR2, and an HCDR3, wherein the amino acid sequences of said HCDR1, HCDR2, and HCDR3 may be as set forth in SEQ ID NO: 21, SEQ ID NO: 23, and SEQ ID NO: 25, respectively.

In this application, said antibody or the antigen-binding fragment thereof may comprise an HCDR1, an HCDR2, and an HCDR3, wherein the amino acid sequences of said HCDR1, HCDR2, and HCDR3 may be as set forth in SEQ ID NO: 21, SEQ ID NO: 24, and SEQ ID NO: 25, respectively.

In this application, said antibody or the antigen-binding fragment that specifically binds to BCMA thereof in the CAR may comprise a light-chain complementarity determining region 3 (LCDR3), and said LCDR3 may comprise an amino acid sequence as set forth in SEQ ID NO: 40: $LQSRX_5FPRX_9$ (SEQ ID NO: 40), wherein $X_5$=I or R, $X_9$=H or T. For example, said sequence can be divided according to Kabat.

In this application, said LCDR3 may comprise an amino acid sequence as set forth in any one of SEQ ID NOs: 15-16.

In this application, said antibody or the antigen-binding fragment thereof may comprise a light-chain complementarity determining region 2 (LCDR2), and said LCDR2 may comprise an amino acid sequence as set forth in SEQ ID NO: 39: $LGX_3NRX_6X_7$ (SEQ ID NO: 39), where $X_3$=R or S, $X_6$=A or P, and $X_7$=A or S. For example, said sequence can be divided according to Kabat.

In this application, said LCDR2 may comprise an amino acid sequence as set forth in any one of SEQ ID NOs: 13-14.

In this application, said antibody or the antigen-binding fragment thereof may comprise a light-chain complementarity determining region 1 (LCDR1), and said LCDR1 may comprise an amino acid sequence as set forth in SEQ ID NO: 38: $RASESVSVX_9GX_{11}X_{12}X_{13}X_{14}H$ (SEQ ID NO: 38), wherein $X_9$=I, P or S, $X_{11}$=A or I, $X_{12}$=H or S, $X_{13}$=F or L, and $X_{14}$=I or L. For example, said sequence can be divided according to Kabat.

In this application, said LCDR1 may comprise an amino acid sequence as set forth in any one of SEQ ID NOs: 10-12.

In this application, said antibody or the antigen-binding fragment thereof may comprise an LCDR1, an LCDR2, and an LCDR3, wherein the amino acid sequences of said LCDR1, LCDR2, and LCDR3 may be as set forth in SEQ ID NO: 38, SEQ ID NO: 39, and SEQ ID NO: 40, respectively.

In this application, said antibody or the antigen-binding fragment thereof may comprise an LCDR1, an LCDR2, and an LCDR3, wherein said LCDR1 may comprise an amino acid sequence as set forth in any one of SEQ ID NOs: 10-12, said LCDR2 may comprise an amino acid sequence as set forth in any one of SEQ ID NOs: 13-14, and said LCDR3 may comprise an amino acid sequence as set forth in any one of SEQ ID NOs: 15-16.

In this application, said antibody or the antigen-binding fragment thereof may comprise an LCDR1, an LCDR2, and an LCDR3, wherein the amino acid sequences of said LCDR1, LCDR2, and LCDR3 may be as set forth in SEQ ID NO: 10, SEQ ID NO: 13, and SEQ ID NO: 15, respectively.

In this application, said antibody or the antigen-binding fragment thereof may comprise an LCDR1, an LCDR2, and an LCDR3, wherein the amino acid sequences of said LCDR1, LCDR2, and LCDR3 may be as set forth in SEQ ID NO: 11, SEQ ID NO: 13, and SEQ ID NO: 16, respectively.

In this application, said antibody or the antigen-binding fragment thereof may comprise an LCDR1, an LCDR2, and an LCDR3, wherein the amino acid sequences of said LCDR1, LCDR2, and LCDR3 may be as set forth in SEQ ID NO: 12, SEQ ID NO: 13, and SEQ ID NO: 16, respectively.

In this application, said antibody or the antigen-binding fragment thereof may comprise an LCDR1, an LCDR2, and an LCDR3, wherein the amino acid sequences of said LCDR1, LCDR2, and LCDR3 may be as set forth in SEQ ID NO: 12, SEQ ID NO: 14, and SEQ ID NO: 16, respectively.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 41, SEQ ID NO: 25, SEQ ID NO: 38, SEQ ID NO: 39, and SEQ ID NO: 40, respectively.

In this application, said antibody or the antigen-binding fragment thereof may comprise an HCDR1, an HCDR2, an HCDR3, an LCDR1, an LCDR2, and an LCDR3, wherein said HCDR1 may comprise an amino acid sequence as set forth in SEQ ID NO: 21, said HCDR2 may comprise an amino acid sequence as set forth in any one of SEQ ID NOs: 22-24, said HCDR3 may comprise an amino acid sequence as set forth in SEQ ID NO: 25, said LCDR1 may comprise an amino acid sequence as set forth in any one of SEQ ID NOs: 10-12, said LCDR2 may comprise an amino acid sequence as set forth in any one of SEQ ID NOs: 13-14, and said LCDR3 may comprise an amino acid sequence as set forth in any one of SEQ ID NOs: 15-16.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 22, SEQ ID NO: 25, SEQ ID NO: 10, SEQ ID NO: 13, and SEQ ID NO: 15, respectively.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 23, SEQ ID NO: 25, SEQ ID NO: 11, SEQ ID NO: 13, and SEQ ID NO: 16, respectively.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 24, SEQ ID NO: 25, SEQ ID NO: 11, SEQ ID NO: 13, and SEQ ID NO: 16, respectively.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 22, SEQ ID NO: 25, SEQ ID NO: 12, SEQ ID NO: 13, and SEQ ID NO: 16, respectively.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 24, SEQ ID NO: 25, SEQ ID NO: 12, SEQ ID NO: 13, and SEQ ID NO: 16, respectively.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 22, SEQ ID NO: 25, SEQ ID NO: 12, SEQ ID NO: 14, and SEQ ID NO: 16, respectively.

In this application, said antibody or the antigen-binding fragment thereof may comprise an HCDR1, an HCDR2, an HCDR3, an LCDR1, an LCDR2, and an LCDR3, wherein the amino acid sequences of said HCDR1, HCDR2, HCDR3, LCDR1, LCDR2, and LCDR3 may be as set forth in SEQ ID NO: 21, SEQ ID NO: 24, SEQ ID NO: 25, SEQ ID NO: 12, SEQ ID NO: 14, and SEQ ID NO: 16, respectively.

In this application, said antibody or the antigen-binding fragment thereof may comprise a frame region H-FR1, a C terminal of said H-FR1 is directly or indirectly linked to an N terminal of said HCDR1, and said H-FR1 may comprise an amino acid sequence as set forth in SEQ ID NO: 42: QVQLUQSGX$_9$EX$_{11}$KX$_{13}$PGASVKVSCKASGYX$_{28}$FA (SEQ ID NO: 42), wherein X$_9$=A or S, X$_{11}$=L or V, X$_{13}$=K or Q, and X$_{28}$=S or T. For example, said sequence can be divided according to Kabat.

In this application, said H-FR1 of said antibody or the antigen-binding fragment thereof may comprise an amino acid sequence as set forth in any one of SEQ ID NOs: 26-29.

In this application, said antibody or the antigen-binding fragment thereof may comprise a frame region H-FR2, and said H-FR2 is located between said HCDR1 and said HCDR2, and may comprise an amino acid sequence as set forth in SEQ ID NO: 43: WVRQAX$_6$GQGLEWX$_{13}$G (SEQ ID NO: 43), wherein X$_6$=P or T, and X$_{13}$=I or M. For example, said sequence can be divided according to Kabat.

In this application, said H-FR2 of said antibody or the antigen-binding fragment thereof may comprise an amino acid sequence as set forth in any one of SEQ ID NOs: 30-32.

In this application, said antibody or the antigen-binding fragment thereof may comprise a frame region H-FR3, and said H-FR3 is located between said HCDR2 and said HCDR3, and may comprise an amino acid sequence as set forth in SEQ ID NO: 44: RFX$_3$FX$_5$LX$_7$TSX$_{10}$STAYLX$_{16}$X$_{17}$SSLX$_{21}$X$_{22}$EDTAVYYCAR (SEQ ID NO: 44), wherein X$_3$=T or V, X$_5$=S or T, X$_7$=D or N, X$_{10}$=A, I or V, X$_{16}$=E or Q, X$_{17}$=I or L, X$_{21}$=K or R, and X$_{22}$=A or S. For example, said sequence can be divided according to Kabat.

In this application, said H-FR3 of said antibody or the antigen-binding fragment thereof may comprise an amino acid sequence as set forth in any one of SEQ ID NOs: 33-36.

In this application, said antibody or the antigen-binding fragment thereof may comprise a frame region H-FR4, an N terminal of said H-FR4 is linked to a C terminal of said HCDR3, and said H-FR4 may comprise an amino acid sequence as set forth in SEQ ID NO: 37: WGQGTLVTVSS (SEQ ID NO: 37). For example, said sequence can be divided according to Kabat.

In this application, said antibody or the antigen-binding fragment thereof may comprise an H-FR1, an H-FR2, an H-FR3, and an H-FR4, wherein the amino acid sequences of said H-FR1, H-FR2, H-FR3, and H-FR4 may be as set forth in SEQ ID NO: 42, SEQ ID NO: 43, SEQ ID NO: 44, and SEQ ID NO: 37, respectively.

In this application, said antibody or the antigen-binding fragment thereof may comprise an H-FR1, an H-FR2, an H-FR3, and an H-FR4, wherein said H-FR1 may comprise an amino acid sequence as set forth in any one of SEQ ID NOs: 26-29, said H-FR2 may comprise an amino acid sequence as set forth in any one of SEQ ID NOs: 30-32, said H-FR3 may comprise an amino acid sequence as set forth in any one of SEQ ID NOs: 33-36, and said H-FR4 may comprise an amino acid sequence as set forth in SEQ ID NO: 37.

In this application, said antibody or the antigen-binding fragment thereof may comprise any set of H-FR1, H-FR2, H-FR3, and H-FR4, selected from the groups consisting of: (1) H-FR1: SEQ ID NO: 26, H-FR2: SEQ ID NO: 30, H-FR3: SEQ ID NO: 33, and H-FR4: SEQ ID NO: 37; (2) H-FR1: SEQ ID NO: 27, H-FR2: SEQ ID NO: 31, H-FR3: SEQ ID NO: 34, and H-FR4: SEQ ID NO: 37; (3) H-FR1: SEQ ID NO: 28, H-FR2: SEQ ID NO: 32, H-FR3: SEQ ID NO: 35, and H-FR4: SEQ ID NO: 37; and (4) H-FR1: SEQ ID NO: 29, H-FR2: SEQ ID NO: 30, H-FR3: SEQ ID NO: 36, and H-FR4: SEQ ID NO: 37.

In this application, said antibody or the antigen-binding fragment thereof may comprise an antibody weight chain variable region (VH), and said VH may comprise an amino acid sequence as set forth in SEQ ID NO: 46: QVQLVQSGX$_9$EX$_{11}$KX$_{13}$PGASVKVSCKASGYX$_{28}$FADYTINWVRQAX$_{41}$GQGLEWX$_{48}$GWINTETREPX$_{59}$YAYDFRX$_{66}$ RFX$_{69}$FX$_{71}$LX$_{73}$TSX$_{76}$STAYLX$_{82}$X$_{83}$SSLX$_{87}$X$_{88}$EDTAV YYCARDIRYVMDYWGQGTLVTVSS (SEQ ID NO: 46), where-in X$_9$=A or S, X$_{11}$=L or V, X$_{13}$=K or Q, X$_{28}$=S or T, X$_{41}$=P or T, X$_{48}$=I or M, X$_{59}$=A or T, X$_{66}$=G or S, X$_{69}$=T or V, X$_{71}$=S or T, X$_{73}$=D or N, X$_{76}$=A, I or V, X$_{82}$=E or Q, X$_{83}$=I or L, X$_{87}$=K or R, and X$_{88}$=A or S. For example, said sequence can be divided according to Kabat.

In this application, said VH of said antibody or the antigen-binding fragment thereof may comprise an amino acid sequence as set forth in any one of SEQ ID NOs: 5-9.

In this application, said antibody or the antigen-binding fragment thereof may comprise a frame region L-FR1, a C terminal of said L-FR1 is directly or indirectly linked to an N terminal of said LCDR1, and said L-FR1 may comprise an amino acid sequence as set forth in SEQ ID NO: 17: EIVLTQSPATLSLSPGERATLSC (SEQ ID NO: 17). For example, said sequence can be divided according to Kabat.

In this application, said antibody or the antigen-binding fragment thereof may comprise a frame region L-FR2, said L-FR2 is located between said LCDR1 and said LCDR2, and said L-FR2 may comprise an amino acid sequence as set forth in SEQ ID NO: 18: WYQQKPGQAPRLLIY (SEQ ID NO: 18). For example, said sequence can be divided according to Kabat.

In this application, said antibody or the antigen-binding fragment thereof may comprise a frame region L-FR3, and said L-FR3 is located between said LCDR2 and said LCDR3, and may comprise an amino acid sequence as set forth in SEQ ID NO: 19: GIPARFSGSGSGTDFTLTISSLEPEDAAIYYC (SEQ ID NO: 19). For example, said sequence can be divided according to Kabat.

In this application, said antibody or the antigen-binding fragment thereof may comprise a frame region L-FR4, an N terminal of said L-FR4 is linked to a C terminal of said LCDR3, and said L-FR4 may comprise an amino acid sequence as set forth in SEQ ID NO: 20: FGQGTKLEIK (SEQ ID NO: 20). For example, said sequence can be divided according to Kabat.

In this application, said antibody or the antigen-binding fragment thereof may comprise an L-FR1, an L-FR2, an L-FR3, and an L-FR4, wherein the amino acid sequences of said L-FR1, L-FR2, L-FR3, and L-FR4 may be as set forth in SEQ ID NO: 17, SEQ ID NO: 18, SEQ ID NO: 19, and SEQ ID NO: 20, respectively.

In this application, said antibody or the antigen-binding fragment thereof may comprise an antibody light chain variable region (VL), and said VL may comprise an amino acid sequence as set forth in SEQ ID NO: 45: EIVLTQSPATLSLSPGERATLSCRASESVSVX$_{32}$GX$_{34}$X$_{35}$X$_{36}$X$_{37}$HWYQQKPGQAPRLL IYLGX$_{56}$NRX$_{59}$X$_{60}$GIPARFSGSGSGTDFTLTISSLEPEDAAIYYCLQSRX$_{97}$FPRX$_{101}$FG QGTKLEIK (SEQ ID NO: 45), wherein X$_{32}$=I, P or S, X$_{34}$=A or I, X$_{35}$=H or S, X$_{36}$=F or L, X$_{37}$=I or L, X$_{56}$=R or S, X$_{59}$=A or P, X$_{60}$=A or S, X$_{97}$=I or R, and X$_{101}$=H or T. For example, said sequence can be divided according to Kabat.

In this application, said VL of said antibody or the antigen-binding fragment thereof may comprise an amino acid sequence as set forth in any one of SEQ ID NOs: 1-4.

In this application, said antibody or the antigen-binding fragment thereof may comprise a VH and a VL, said VH may comprise an amino acid sequence as set forth in SEQ ID NO: 46, and said VL may comprise an amino acid sequence as set forth in SEQ ID NO: 45.

In this application, said antibody or the antigen-binding fragment thereof may comprise a VH and a VL, said VH may comprise an amino acid sequence as set forth in any one of SEQ ID NOs: 5-9, and said VL may comprise an amino acid sequence as set forth in any one of SEQ ID NOs: 1-4.

In this application, said antibody or the antigen-binding fragment thereof may comprise a VH and a VL, said VH may comprise an amino acid sequence as set forth in SEQ ID NO: 5, and said VL may comprise an amino acid sequence as set forth in SEQ ID NO: 1.

In this application, said antibody or the antigen-binding fragment thereof may comprise a VH and a VL, said VH may comprise an amino acid sequence as set forth in SEQ ID NO: 6, and said VL may comprise an amino acid sequence as set forth in SEQ ID NO: 2.

In this application, said antibody or the antigen-binding fragment thereof may comprise a VH and a VL, said VH may comprise an amino acid sequence as set forth in SEQ ID NO: 7, and said VL may comprise an amino acid sequence as set forth in SEQ ID NO: 2.

In this application, said antibody or the antigen-binding fragment thereof may comprise a VH and a VL, said VH may comprise an amino acid sequence as set forth in SEQ ID NO: 8, and said VL may comprise an amino acid sequence as set forth in SEQ ID NO: 2.

In this application, said antibody or the antigen-binding fragment thereof may comprise a VH and a VL, said VH may comprise an amino acid sequence as set forth in SEQ ID NO: 9, and said VL may comprise an amino acid sequence as set forth in SEQ ID NO: 3.

In this application, said antibody or the antigen-binding fragment thereof may comprise a VH and a VL, said VH may comprise an amino acid sequence as set forth in SEQ ID NO: 8, and said VL may comprise an amino acid sequence as set forth in SEQ ID NO: 3.

In this application, said antibody or the antigen-binding fragment thereof may comprise a VH and a VL, said VH may comprise an amino acid sequence as set forth in SEQ ID NO: 9, and said VL may comprise an amino acid sequence as set forth in SEQ ID NO: 4.

In this application, said antibody or the antigen-binding fragment thereof may comprise a VH and a VL, said VH may comprise an amino acid sequence as set forth in SEQ ID NO: 8, and said VL may comprise an amino acid sequence as set forth in SEQ ID NO: 4.

In this application, said antibody or the antigen-binding fragment thereof may comprise a scFv, and said scFv may comprise said VH and said VL. In said scFv, the C terminal of said VH and the N terminal of said VL may be linked directly, for example, in-frame. In said scFv, the C terminal of said VH and the N terminal of said VL may be linked indirectly, for example, by a linker (e.g., a linker peptide). In said scFv, the C terminal of said VL and the N terminal of said VH may be linked directly, for example, in-frame. In said scFv, the C terminal of said VL and the N terminal of said VH may be linked indirectly, for example, by a linker (e.g., a linker peptide).

In this application, the BCMA domain of the CAR may comprise a VH, a VL and a linker peptide, said VH may comprise an amino acid sequence as set forth in SEQ ID NO: 5, said VL may comprise an amino acid sequence as set forth in SEQ ID NO: 1, and said linker peptide may comprise an amino acid sequence as set forth in SEQ ID NO: 58.

In this application, the BCMA domain of the CAR may comprise a VH, a VL and a linker peptide, said VH may comprise an amino acid sequence as set forth in SEQ ID NO: 6, said VL may comprise an amino acid sequence as set forth in SEQ ID NO: 2, and said linker peptide may comprise an amino acid sequence as set forth in SEQ ID NO: 58.

In this application, the BCMA domain of the CAR may comprise a VH, a VL and a linker peptide, said VH may comprise an amino acid sequence as set forth in SEQ ID NO: 7, said VL may comprise an amino acid sequence as set forth in SEQ ID NO: 2, and said linker peptide may comprise an amino acid sequence as set forth in SEQ ID NO: 58.

In this application, the BCMA domain of the CAR may comprise a VH, a VL and a linker peptide, said VH may comprise an amino acid sequence as set forth in SEQ ID NO: 8, said VL may comprise an amino acid sequence as set forth in SEQ ID NO: 2, and said linker peptide may comprise an amino acid sequence as set forth in SEQ ID NO: 58.

In this application, the BCMA domain of the CAR may comprise a VH, a VL and a linker peptide, said VH may comprise an amino acid sequence as set forth in SEQ ID NO: 9, said VL may comprise an amino acid sequence as set forth in SEQ ID NO: 3, and said linker peptide may comprise an amino acid sequence as set forth in SEQ ID NO: 58.

In this application, the BCMA domain of the CAR may comprise a VH, a VL and a linker peptide, said VH may comprise an amino acid sequence as set forth in SEQ ID NO: 8, said VL may comprise an amino acid sequence as set forth in SEQ ID NO: 3, and said linker peptide may comprise an amino acid sequence as set forth in SEQ ID NO: 58.

In this application, the BCMA domain of the CAR may comprise a VH, a VL and a linker peptide, said VH may comprise an amino acid sequence as set forth in SEQ ID NO: 9, said VL may comprise an amino acid sequence as set forth in SEQ ID NO: 4, and said linker peptide may comprise an amino acid sequence as set forth in SEQ ID NO: 58.

In this application, the BCMA domain of the CAR may comprise a VH, a VL and a linker peptide, said VH may comprise an amino acid sequence as set forth in SEQ ID NO: 8, said VL may comprise an amino acid sequence as set forth in SEQ ID NO: 4, and said linker peptide may comprise an amino acid sequence as set forth in SEQ ID NO: 58.

In this application, the BCMA domain of the CAR may comprise a VH, a VL and a linker peptide, the C terminal of said VL may be linked to the N terminal of the VH by a linker peptide, and the linker peptide may comprise an amino acid sequence as set forth in SEQ ID NO: 58.

In this application, the BCMA domain of the CAR may comprise a scFv, and said scFv may comprise an amino acid sequence as set forth in any one of SEQ ID Nos: 39-49. In this application, said scFv may comprise an amino acid sequence at least 80% (e.g., at least 85%, 90%, 91%, 92%, 93%, 94%, 95%, 96%, 97%, 98%, 99% or higher) identical to the amino acid sequence as set forth in any one of SEQ ID NOs: 39-49.

Chimeric antigen receptor (CAR)

In this application, the CAR may further comprise an intracellular domain in addition to the extracellular BCMA domain.

In some cases, the CAR may comprise an intracellular costimulatory signaling domain that can provide a stimulatory signal. Said costimulatory signaling domain may include, but be not limited to, costimulatory molecules composed of costimulatory signaling regions in the following group consisting of CD137, CD28, 4-1BB, OX-40, and ICOS, and combinations thereof. For example, said costimulatory domain may be a CD137-derived stimulatory domain. For example, said costimulatory domain may comprise an amino acid sequence as set forth in SEQ ID NO: 62.

In some cases, the CAR may comprise an intracellular signaling domain, which may comprise a domain containing at least one ITAM. Said intracellular signaling domain can transmit an activation signal into the cell. An exemplary signaling domain may be signaling domains derived from proteins elected from the group consisting of, but not limited to, CD3$\zeta$, CD3$\delta$, CD3$\gamma$, CD3$\epsilon$, CD79a, CD79b, Fc$\epsilon$RI$\gamma$, Fc$\epsilon$RI$\beta$, FcyRIIa, bovine leukemia virus gp30 activation region, Epstein-Barr virus (EBV) LMP2A, simian immunodeficiency virus PBj 14Nef, Kaposi's sarcoma-associated herpesvirus (HSKV), DAP10 and DAP-12, and variants thereof. For example, said intracellular signaling domain may be a CD3$\zeta$-derived signaling domain. For example, said intracellular signaling domain may comprise an amino acid sequence as set forth in SEQ ID NO: 63.

In some cases, the CAR may comprise a transmembrane domain which is a sequence in a cell surface protein that spans the cell membrane. The transmembrane domain may comprise a hydrophobic alpha helix. The transmembrane domain may be derived from CD28 and has good stability. The transmembrane domain may be derived from any type I transmembrane protein. The ansmembrane domain may be a synthetic sequence predicted to form a hydrophobic helix. Said transmembrane domain may comprise transmembrane domains derived from one or more proteins selected from the group consisting of: CD28, CD3e, CD45, CD4, CD5, CD8, CD9, CD16, CD22, CD33, CD37, CD64, CD80, CD86, CD134, CD137, and CD154. For example, said transmembrane domain may be a CD8-derived transmembrane domain. For example, said transmembrane domain may comprise an amino acid sequence as set forth in SEQ ID NO: 61.

In some cases, the CAR may comprise a hinge region, which may be located between said extracellular targeting moiety and said transmembrane domain. Said hinge region may comprise a hinge region derived from one or more proteins selected from the group consisting of: CD28, IgG1, IgG4, IgD, 4-1BB, CD4, CD27, CD7, CD8 alpha, PD-1, ICOS, OX40, NKG2D, NKG2C, Fc$\epsilon$RI$\gamma$, BTLA, GITR, DAP10, CD40L, TIM1, CD226, SLAM, CD30, and LIGHT. For example, said hinge region may be derived from CD8. For example, said hinge region may comprise an amino acid sequence as set forth in SEQ ID NO: 60.

In this application, the CAR may further comprise a signal peptide, for example, human CD8-derived signal peptide, at the N terminal of the BCMA domain. For example, the signal peptide may comprise an amino acid sequence as set forth in SEQ ID NO: 59.

In this application, from the N terminal to the C terminal, the CAR may sequentially comprise a BCMA targeting moiety (e.g., said antigen-binding protein, e.g., said scFv), said hinge region, said transmembrane domain, said costimulatory signaling domain, and said signaling domain.

In this application, from the N terminal to the C terminal, the CAR may sequentially comprise said scFv, said CD8-derived hinge region, said CD8-derived transmembrane domain, said CD137-derived costimulatory signaling domain and said CD3$\zeta$-derived signaling domain. For example, the CAR may comprise an amino acid sequence as set forth in any one of SEQ ID NOs: 64-71.

In this application, the C terminal of the CAR may be linked to a leptin and/or a functional fragment thereof, and/or low-density lipoprotein receptor-related protein or a fragment thereof. For example, said leptin may comprise secretory leptins. For example, low-density lipoprotein receptor-related protein 6.

In this application, said CAR, said leptin and/or the functional fragment thereof, and/or said low-density lipoprotein receptor-related protein or the fragment thereof may be linked by a self-cleaving peptide (e.g., a 2A peptide such as T2A, P2A, and E2A).

In this application, the C terminal of the CAR may be linked to L6-Li. For example, said L6-Li may comprise an amino acid sequence as set forth in SEQ ID NO: 107. For example, the nucleic acid molecule encoding said L6-Li may comprise a nucleotide sequence as set forth in SEQ ID NO: 106.

In this application, from the N terminal to the C terminal, the CAR may sequentially comprise a BCMA targeting moiety (e.g., said antigen-binding protein, e.g., said scFv), said hinge region, said transmembrane domain, said costimulatory signaling domain, said signaling domain, and said L6-Li.

For example, from the N terminal to the C terminal, the CAR may sequentially comprise said scFv, said CD8-derived hinge region, said CD8-derived transmembrane domain, said CD137-derived costimulatory signaling domain, said CD3$\zeta$-derived signaling domain, and said L6-Li. For example, the CAR may comprise an amino acid sequence as set forth in SEQ ID NO: 74.

Nucleic acid molecules, vectors, cells, preparation methods and pharmaceutical compositions

In another aspect, this application further provides one or more isolated nucleic acid molecules that may encode the chimeric antigen receptor (CAR) of this application. The one or more isolated nucleic acid molecules of this application may be an isolated form of nucleotides, deoxyribonucleotides or ribonucleotides of any length, or analogs isolated from their natural environment or artificially synthesized, but can encode the CAR of this application. For example, the nucleic acid molecule may comprise a nucleotide sequence as set forth in any one of SEQ ID NOs: 95-102 and 105.

In another aspect, this application further provides a vector, which may comprise the nucleic acid molecule described herein. The vector can be expressed by transforming, transducing or transfecting a host cell so that the genetic material elements carried thereon can be expressed in the host cell. For example, the vector may include: plasmids; phagemids; cosmids; artificial chromosomes such as yeast artificial chromosomes (YACs), bacterial artificial chromosomes (BACs) or P1-derived artificial chromosomes (PACs); phages such as λ phage or M13 phage and animal viruses, etc. Animal virus species used as vectors include retroviruses (including lentiviruses), adenoviruses, adeno-associated viruses, herpesviruses (such as herpes simplex virus), poxviruses, baculoviruses, papillomaviruses, and papovavirus (e.g., SV40). For another example, said vector may comprise a variety of elements that control expression, including promoter sequence, transcriptional initiation sequence, enhancer sequence, selective element and reporter gene. In addition, said vector may also comprise replication initiation sites. In addition, said vector may also comprise components that assist its entry into cells, such as, but not exclusively, viral particles, liposomes, or protein coats.

In another aspect, this application further provides an immune effector cell that may comprise the CAR, the nucleic acid molecule, and/or the vector of this application. Said cell may include a progeny of an individual cell. Due to natural, accidental or deliberate mutations, the progeny cell may not be necessarily identical (either in the morphology of the total DNA complement or in the genome) to the original parent cell. In certain embodiments, said cell may further include cells transfected in vitro with vector of this application. In certain embodiments, said cell may be a mammalian cell. In certain embodiments, said immune effector cell includes T lymphocytes, for example, α/β T lymphocytes and γ/δ T lymphocytes. In certain embodiments, said immune effector cell may include natural killer (NK) cells, natural killer T (NKT) cells, mast cells and bone marrow-derived phagocytes.

In one aspect, this application provides a modified immune effector cell, comprising a chimeric antigen receptor and/or a coding element thereof, or comprising a T cell receptor and/or a coding element thereof, and the modified immune cell further comprises: a leptin and/or a functional fragment thereof; and/or a leptin receptor and/or a functional fragment thereof. Compared with immune cells without corresponding modification, the modified immune cell has increased expression of said leptin receptor and/or the functional fragment thereof. In this application, the immune effector cell may comprise leptin and/or a functional fragment thereof, and/or a leptin receptor and/or a functional fragment thereof, and a low-density lipoprotein receptor-related protein or a fragment thereof. For the modification of immune effector cells, please refer to WO2021057932A1. The description in WO2021057932A1 can also be applied to this application.

In another aspect, this application further provides a method for preparing the immune effector cell described herein. The method may include: introducing the isolated nucleic acid molecule of this disclosure or the vector of this application into the immune effector cell.

In another aspect, this application further provides a composition, which may comprise the immune effector cell described herein. In certain embodiments, the composition may further comprise optionally a pharmaceutically acceptable carrier. In certain embodiments, the acceptable ingredients of the composition are not toxic to recipients at the doses and concentrations used. The pharmaceutical composition of this application includes, but is not limited to, liquid, frozen and lyophilized compositions. In certain embodiments, said pharmaceutically acceptable adjuvant may include any and all solvents, dispersion media, isotonic agents, and absorption delaying agents that are compatible with said immune effector cell, are generally safe and nontoxic, and are neither biologically nor otherwise undesirable.

In certain embodiments, the composition may be administered by routes including parenteral, transdermal, intracavity, intraarterial, intrathecal and/or intranasal administration or may be directly injected into tissues. For example, the composition may be administered to a patient or subject by infusion or injection. In certain embodiments, the pharmaceutical composition may be administered by different means, such as intravenous, intraperitoneal, subcutaneous, intramuscular, topical or intradermal administration.

Treatment method

In another aspect, this application further provides a use of the chimeric antigen receptor of this application, the nucleic acid molecule of this application, the vector of this application, the immune effector cell of this application and/or the composition of this application in the manufacture of a medicament, wherein said medicament is used for treating a disease or disorder associated with BCMA expression.

In this application, the disease or disorder associated with BCMA expression is a cancer or a malignant tumor. For example, the disease or disorder associated with BCMA expression may include BCMA-positive tumors. For example, said cancer or malignant tumor may include solid tumors or non-solid tumors. For example, said cancer or malignant tumor may be a non-solid tumor. For example, said cancer or malignant tumor may include myeloma. for example, said myeloma may be multiple myeloma.

In another aspect, this application further provides a method for preventing, alleviating or treating a tumor. The method may comprise: administering the CAR, the nucleic acid molecule, the vector, the immune effector cell and/or the pharmaceutical composition of this application to a subject in need. In this application, the administration may be carried out by different means, such as intravenous, intratumoral, intraperitoneal, subcutaneous, intramuscular, topical or intradermal administration.

In this application, said subject may include humans and non-human animals. For example, said subject may include, but is not limited to, cats, dogs, horses, pigs, cows, sheep, rabbits, mice, rats or monkeys. Without wishing to be bound by any theory, the following examples are only for illustrating various technical solutions of the invention of this application, but are not intended to limit the scope of the invention of this application.

## EXAMPLES

### Example 1 Production of scFv

Recombinant human BCMA-hFc and BCMA-His proteins, prepared by Oricell Therapeutics Co.,Ltd., were alternately sorted with the natural human phage antibody library for a total of four rounds, where the human BCMA-hFc represents a protein formed by linking human BCMA to human Fc, the amino acid sequence of human BCMA protein (GenBank ID: BAB60895.1) can be found in NCBI's Official website, and the amino acid sequence of human Fc is as set forth in SEQ ID NO: 57. After four rounds of sorting, significant enrichment was observed. Phage antibody clones obtained by sorting were identified using ELISA. The obtained positive antibody sequence was then subjected to affinity testing and sequencing identification. The sorted candidate antibodies were subjected to one to two rounds of affinity evolution and also subjected to affinity testing and binding activity identification by flow cytometry again, and a total of eight phage antibody clones that can specifically bind to human BCMA were obtained and named hBCMA-01, hBCMA-02, hBCMA-15, hBCMA-17, hBCMA-19, hBCMA-20, hBCMA-22, and hBCMA-23, respectively. After sequencing, the nucleic acid sequences of their VL and VH were obtained. The nucleic acid sequences of the VL and the VH were linked by a linker peptide nucleic acid sequence having a nucleic acid sequence as set forth in SEQ ID NO: 58 and an amino acid sequence as set forth in SEQ ID NO: 85 to form a single-chain antibody (scFv). The scFv sequence of the positive control was huBCMA-10 disclosed in Patent Application US 2017/0226216 A1. The nucleic acid sequences of all the above nine scFvs (including huBCMA-10) were delivered to GenScript, Nanjing for gene synthesis, and a forward primer having a nucleic acid sequence as set forth in SEQ ID NO: 87 was added to the 5' terminal of the scFv nucleic acid sequence and a reverse primer having a nucleic acid sequence as set forth in SEQ ID NO: 88 was added to the 3' terminal of the scFv nucleic acid sequence for molecular construction in the next step of homologous recombination. The VH and VL gene sequences of these eight antibody clones and the positive control are shown in Table 1.

Table 1. VH and VL sequences of eight antibody clones and the positive control

| scFv sequence | Nucleic acid sequence (SEQ ID NO:) | | Amino acid sequence (SEQ ID NO:) | |
| --- | --- | --- | --- | --- |
| | VL | VH | VL | VH |
| hBCMA-01 | 75 | 80 | 1 | 5 |
| hBCMA-02 | 76 | 81 | 2 | 6 |
| hBCMA-15 | 76 | 82 | 2 | 7 |
| hBCMA-17 | 76 | 83 | 2 | 8 |
| hBCMA-19 | 77 | 84 | 3 | 9 |
| hBCMA-20 | 77 | 83 | 3 | 8 |
| hBCMA-22 | 78 | 84 | 4 | 9 |
| hBCMA-23 | 78 | 83 | 4 | 8 |
| huBCMA-10 | 79 | 85 | 47 | 48 |

### Example 2 Construction of CAR lentivirus vectors

The CAR lentivirus core empty plasmid (hereinafter referred to as CAR lentivirus empty vector, constructed by Oricell Therapeutics Co.,Ltd., or CAR lentivirus vector (comprising the L6-Li element, please refer to Patent Application Publication No. WO2021057932A1; the amino acid sequence of L6-Li is as set forth in SEQ ID NO: 107) (hereinafter referred to as CAR-L6-Li lentivirus vector, constructed by OriCell Therapeutics, Shanghai) was double-digested by using SphI and NotI endonuclease (purchased from NEB). The former produced a 7749bp linearized fragment, and the latter produced a 9605bp linearized fragment.

After gel cutting and recovery, the produced 7749 bp linearized fragment and the nucleic acid sequence fragment of the scFv (including homology arms at both ends) obtained in Example 1 were mixed in a molar ratio of 1:3 (not more than 10 μl in volume), and after homologous recombination, E. coli DH5α competent cell was transformed. Single colonies were picked, shaken, plasmid extracted and sent for sequencing to verify whether the inserted scFv sequence was correct.

After gel cutting and recovery, the produced 9605 bp linearized fragment and the nucleic acid sequence fragments of the scFvs in hBCMA-22 and huBCMA-10 (including homology arms at both ends) obtained in Example 1 were mixed in a molar ratio of 1:3 (not more than 10 μl in volume), and after homologous recombination, E. coli DH5α competent cell was transformed. Single colonies were picked, shaken, plasmid extracted and sent for sequencing identification.

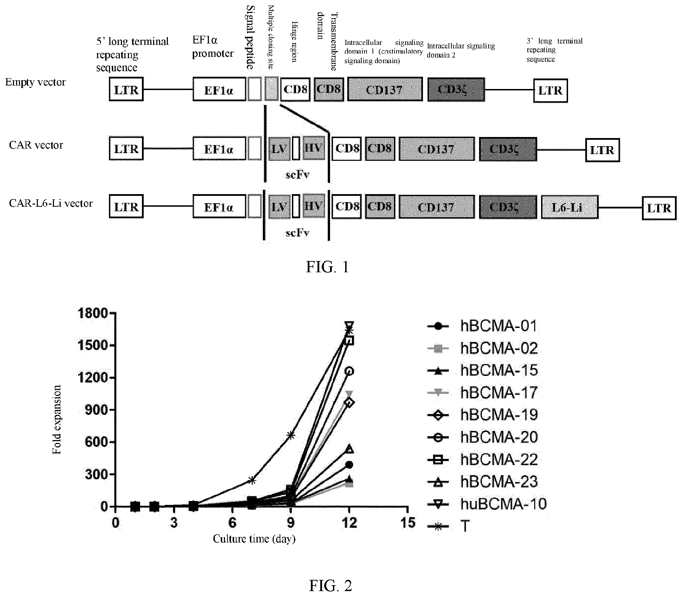

The CAR and various elements of CAR-L6-Li in the CAR lentivirus empty vector and h(u)BCMA-XX core plasmid (hereinafter referred to as h(u)BCMA-XX core plasmid) and connection sequences are shown in FIG. 1. The amino acid sequences and nucleic acid sequences of the various elements are shown in Table 2. The CAR portion of the CAR lentivirus empty vector comprises the signal peptide-CD8-derived hinge region-CD8-derived transmembrane domain-CD137-derived costimulatory domain-CD3ζ-derived intracellular signaling domain in sequence. Based on the CAR lentivirus empty vector, the CAR vector has a scFv targeting BCMA inserted between the signal peptide and the CD8-derived hinge region. Based on the CAR vector, the CAR-L6-Li vector has a L6-Li sequence linked to the 3' terminal of CD3ζ. The 5' terminal of each vector is linked to the EF1α promoter. The sequences of the CAR portions in the plasmid vectors are shown in Table 3.

Table 2 Sequences of various elements in CAR and CAR-L6-Li

| Element | Nucleotide sequence (SEQ ID NO) | Amino acid sequence (SEQ ID NO) |
|---|---|---|
| Signal peptide | 90 | 59 |
| CD8-derived hinge region | 91 | 60 |
| CD8-derived transmembrane domain | 92 | 61 |
| CD 13 7-derived costimulatory domain | 93 | 62 |
| CD3ζ-derived intracellular signaling domain | 94 | 63 |
| L6-Li | SEQ ID NO: 106 | SEQ ID NO: 107 |

Table 3 Sequences of CAR portions in plasmid vectors

| | Nucleotide sequence (SEQ ID NO) | Amino acid sequence (SEQ ID NO) |
|---|---|---|
| hBCMA-01 CAR | 95 | 64 |
| hBCMA-02 CAR | 96 | 65 |
| hBCMA-15 CAR | 97 | 66 |
| hBCMA-17 CAR | 98 | 67 |
| hBCMA-19 CAR | 99 | 68 |
| hBCMA-20 CAR | 100 | 69 |
| hBCMA-22 CAR | 101 | 70 |
| hBCMA-23 CAR | 102 | 71 |
| huBCMA-10 CAR | 103 | 72 |
| huBCMA-10-L6-Li CAR | 104 | 73 |
| huBCMA-22-L6-Li CAR | 105 | 74 |

**Example 3 Lentivirus packaging**

As an example, the system used to construct the lentivirus vector of the present invention was the third generation. The system was composed of three plasmids, i.e., a packaging plasmid psPAX2 (Addgene, plasmid number #12260) encoding Gag-Pol protein and Rev protein, a PMD2.G plasmid (Addgene, plasmid number #12259) encoding an evelope protein VSV-G (Addgene, plasmid number #12259), and a core plasmid, i.e., the CAR lentivirus plasmids containing the scFv sequence in the above Examples 1-2 (i.e., hBCMA-01, hBCMA-02, hBCMA-15, hBCMA-17, hBCMA-19, hBCMA-20, hBCMA-22, hBCMA-23, and huBCMA-10). The expression of the CAR gene in each CAR lentivirus plasmid was controlled by the elongation factor-1a (EF-1$\alpha$) promoter.

The packaging process of lentivirus was as follows:

(1) About $2.5 \times 10^6$ 293T cells in good growth status (generally, cells with passage number less than 20 were selected) were added into 5 ml of 293T cell complete culture medium (high-sugar DMEM containing 10% FBS) and well mixed and then inoculated into a 6 cm culture dish (20-24 hours later, the cell confluence was required to reach 80-90%) and incubated overnight in a 37 °C, 5% $CO_2$ incubator.

(2) About 20-24 hours later, a total of 6 $\mu$g of three plasmids (the mass ratio of CAR lentivirus core plasmid: psPAX2: PMD2.G=4:3:2) were added into 300 $\mu$L of Opti-MEM medium and mixed and 18 uL of a FuGENEHD transfection reagent (Promega, E2311) was then added and well mixed carefully. The mixed solution was set still at room temperature for 15-20 minutes, and then carefully added dropwise into a 6 cm culture dish and shaken carefully. The cells were incubated overnight in a 37 °C, 5% $CO_2$ incubator.

(3) About 20-24 hours after transfection, the upper liquid was carefully removed from the 6 cm culture dish, and 5 mL of 293T cell complete culture medium was then added slowly.

(4) About 48 hours after transfection, the upper liquid (containing dead cells and debris) in the 6 cm culture dish was harvested and centrifuged at 3000 g for 5 minutes. The upper virus liquid with the cell debris removed was transferred to a new centrifuge tube, aliquoted and stored at - 80 °C for later use.

**Example 4 Determination of virus titer**

Lentivirus titer was determined as follows:

(1) The virus liquid prepared in Example 3 was thawed quickly.

(2) 293T cells in good growth status (generally, cells with passage number less than 20) were taken, the upper waste liquid was discarded, the cells were washed with PBS and digested with 0.25% trypsin (GIBICO) at 37 °C for about 3 minutes. After the cells were completely digested, a certain volume of 293T cell complete culture medium was added to stop the reaction, and samples were taken for cell counting. The cell density was then adjusted to $2.0 \times 10^5$ cells/mL, and polybrene with a final concentration of 10 $\mu$g/mL was added, and then the cells were inoculated in six-well plates at 2.5 mL/well.

(3) The above virus liquid was then added to the six-well plates respectively. Generally, three loading gradients, i.e., 2 $\mu$L, 5 $\mu$L and 10 $\mu$L, are made for each virus liquid, and one to two blank wells without the virus liquid need to be set up as negative control for flow cytometry.

(4) The cell culture plates were placed in a 37 °C, 5% $CO_2$ incubator for static culture.

(5) After the cells were cultured statically for 48 hours, the upper waste liquid was discarded. The cells were washed with PBS and then digested with 0.25% trypsin (GIBICO) at 37 °C for about 3 minutes. After the cells were completely digested, a certain volume of 293T cell complete culture medium was added to stop the reaction, and samples were taken for cell counting. About $5 \times 10^5$ cells were then taken and placed in each 1.5 mL sterile centrifuge tube and marked.

(6) NBS solution (PBS solution containing 1% newborn calf serum) was added to make up to 1.2-1.5 mL. The mixed solution was then centrifuged at 4 °C, 500 g for 5 minutes and the supernatant was then discarded.

(7) 1 mL of NBS was added into each tube, and the mixed solution was gently pipetted and well mixed, and then centrifuged at 4 °C, 500 g for 5 minutes, and the supernatant was then discarded.

(8) 50 $\mu$l of NBS and 0.5 $\mu$g of BCMA-Fc-Avi protein (Aero, BC7-H82FO) were added into each tube. The mixed solution was gently pipetted with a pipette and well mixed (no primary antibody was added to the negative control), and incubated at 4 °C for 60 minutes.

(9) After incubation, 1 ml of NBS was directly added to resuspend the cells, the cell resuspension was then centrifuged at 4 °C, 500 g for 5 minutes, and the supernatant was discarded.

(10) 50 $\mu$l of NBS and 0.5 $\mu$l of Native Streptavidin protein (DyLight® 650) (Abcam, ab134341) were added into each tube (no Native Streptavidin protein was added to the negative control), and then incubated at 4 °C for 30 minutes.

(11) Step (9) was performed again.

(12) 200 $\mu$l of NBS-resuspended cells were added into each tube.

(13) The fluorescence expression percentage was detected on a flow cytometer BD FACS CantoII, and the fluorescence was Dylight 650.

(14) Data processing: The results are usable only when the positive rate of test tube minus the positive rate of negative control is within a range of 5% to 20%.

$$\text{Lentivirus titer (IU/mL)} = \text{number of cells on plate} \times (\text{positive rate of test tube}$$

$$- \text{positive rate of control tube}) / \text{volume of inoculated virus liquid}$$

After testing, the titer of each of the above scFv-containing CAR viruses ranged from $5 \times 10^6$ IU/mL to $15 \times 10^6$ IU/mL.

**Example 5 Infection of T cell and *in vitro* expansion of BCMA CAR-T cells**

The CAR-T cell containing the BCMA scFv sequence was prepared as follows:

(1) Human peripheral blood mononuclear cells (PBMC) were isolated, by Ficoll density gradient centrifugation, from blood samples collected by an apheresis machine (apheresis blood was provided by volunteers of the Ark Project initiated by Oricell Therapeutics Co., Ltd.).

(2) After PBMC was sorted by CD3-positive magnetic beads, CD3+T cells with a purity >90% were obtained. Please refer to the product manual (MACS, DS130-050-101) for the specific method of T sorting.

(3) The CD3+T cells were resuspended with a T cell complete culture medium that was prepared by sequentially adding 5% FBS and 300 IU/mL interleukin 2 (125Ala ) to the (X-VIVO 15 (Lonza) medium). Washed CD3/CD28 Dynabeads (Gibco, 40203D) were added at 3 times the amount of cells, T cell complete culture medium was added again, the cell density was adjusted to $1.0\text{-}1.2\times10^6$ cells/mL, and the cells were placed in a 37 °C, 5% $CO_2$ incubator for activation and culture (denoted as D0).

(4) Generally, lentiviral transduction was performed 20-24 hours after T cell activation.

(5) T cells that have been activated for 20-24 hours were collected, centrifuged at 500 g for 5 minutes, and resuspended in a certain volume of T cell complete culture medium and then sampled and counted. According to the ratio of the virus multiplicity of infection (MOI) of 3-6, the virus liquid having the titer determined in Example 3 was added respectively, and polybrene was then added till a final concentration of 5 $\mu$g/ml. Next, the T cell complete culture medium was added again, the cell density was adjusted to $0.6\text{-}1.0\times10^6$ cells/mL, and the cells were then incubated at 37 °C, 5% $CO_2$ incubator. Another portion of T cells without lentiviral infection was used as a negative control group.

(6) About 20-24 hours after viral transduction, T cells were collected from each group, centrifuged at 500 g for 5 minutes, resuspended in a certain volume of T cell complete medium, and then sampled and counted. The T cell complete culture medium was added again and the cell density was adjusted to $0.5\text{-}0.7\times10^6$ cells/mL

(7) Then, CAR-T cells were counted everyday or every two days, and the T cell complete culture medium was added again according to the actual situation, and the cell density was adjusted to $0.5\text{-}1.0\times10^6$ cells/mL.

(8) Generally, Dynabeads were removed on D7 of culture, and the total culture period was about 12 days.

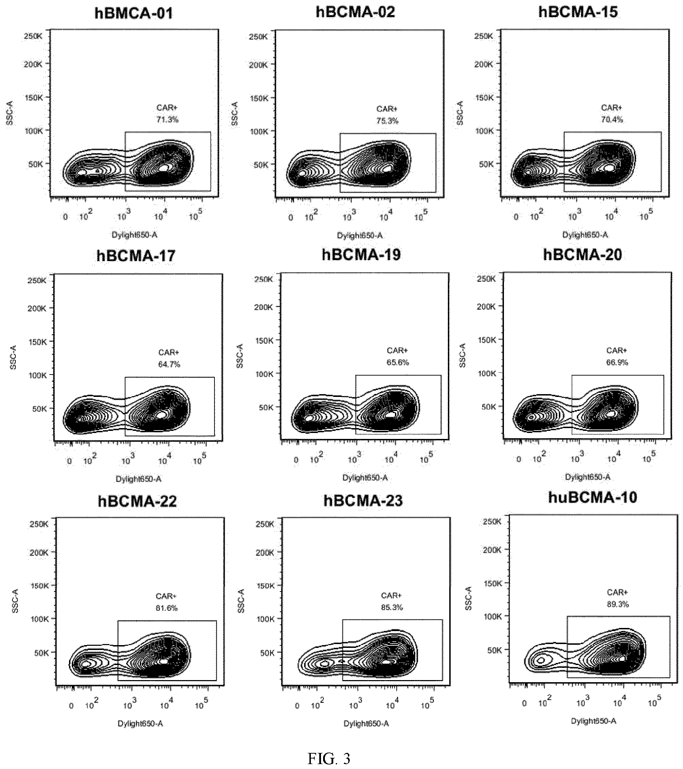

As shown in FIGS. 2 and 3, the total expansion fold of BCMACAR-T cells after activation and culture for 12 days ranges from 100 to 1600, and the CAR positivity rate after infection ranges from 65% to 90%. All can be used for cytological functional experiments.

**Example 6 Evaluation on *in vitro* targeted killing activity of CAR-T cells**

The MM1s cell is a myeloma cell that expresses BCMA, and the K562 cell is a tumor cell that does not express BCMA. In this test, we first constructed these two tumor cells that stably express Luciferase, and then co-incubated with the CAR-T cell for a period of time, and then the activity of the Luciferase enzyme was tested through the substrate on the basis of a principle which was detailed in Promega, E2510, to determine the targeted killing ability of the CAR-T cell.

MM1s-Luci and K562-Luci that overexpress Luciferase were constructed as follows:

(1) The pGL4.30[luc2P/NFAT-RE/Hygro] Vector was purchased from Promega Company, the luc2P reporter gene (228-2003) in the vector was cloned into the lentivirus core empty plasmid without the CAR structure (the empty vector contains the GFP reporter gene), and the resulting plasmid was defined as Luciferase-GFP lentivirus core plasmid.

(2) The Luciferase-GFP (Luci-GFP) lentivirus was packaged according to the method stated in Example 3, and the titer of the Luciferase-GFP lentivirus was tested through a GFP fluorescence channel with reference to the method stated in Example 4.

(3) According to MOI=5-10, the MM1s cell and the K562 cell were infected respectively. After stable culture for 3-4 weeks, the GFP+ cell colonies were sorted through a flow sorter to construct MM1s-Luci-GFP cell and K562-Luci-GFP cell.

The in vitro killing activity of the CAR-T cell was tested during the culture period of D8 to D12. The CAR positivity rate of each group was required to be tested in advance and 0.5-1.0×10⁶ cells were taken. For the testing method, please refer to steps (6)-(13) in Example 4.

The cell infected with CAR lentivirus was an effector cell, the T cell not infected with lentivirus was a blank effector cell, and the MM1s-Luci-GFP cell and the K562-Luci-GFP were target cells.

The testing process of in vitro killing activity of CAR-T cell was as follows:

(1) The CAR positivity rate of each group was tested by flow cytometry one day in advance.
(2) On the day of the killing test, the CAR positive rates of all the groups were adjusted to be consistent by adding blank effector cells, and the medium was CAR-T medium without cytokines.
(3) Killing activity testing was carried out in 96-well plates. Three effector-target ratios of 3:1, 1:1 and 0.3:1 (or two effector-target ratios of 3:1 and 1:1) were set. The number of target cells in each 96-well plate was fixed at $2\times10^4$. The target cell and the effector cell were added in sequence. Three replicate wells were set at each effector-to-target ratio for each group, and it was required to set another three separate target cell wells as negative controls.
(4) Generally, the killing activity was tested 5-6 hours after killing. That is, the cell suspension in the 96-well plates was first pipetted and well mixed, 1/2 volume of the cell suspension was taken evenly and added into completely blank 96-well plates, an equal volume of Luciferase substrate was then added, and the cell solution was then kept at room temperature for 10-15 minutes in the dark. The corresponding fluorescence value was then read on the ELIASA. For the specific testing method, please refer to Steady-Glo® Luciferase Detection System (Promega, E2510).
(5) Since only viable MM1s cells can be detected, the killing toxicity of the CAR-T cell against the target cell can be calculated as follows:

$$\text{Killing toxicity } (\%) = \frac{\text{Fluorescence value}_{\text{target}} - \text{Fluorescence value}_{\text{CAR-T+target}}}{\text{Fluorescence value}_{\text{targe}}} \times 100\%$$

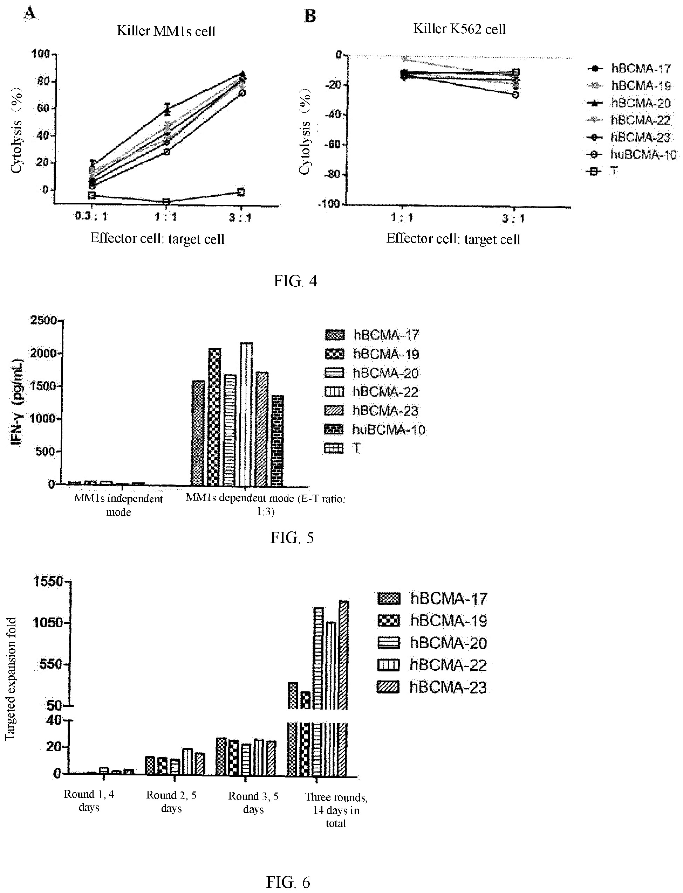

As shown in FIGS. 4A and 4B, compared with blank effector cell T cell, the BCMACAR-T cells of all groups have strong killing activity against BCMA-expressing MM1s cells and also show obvious dependence on the effector-target ratio, but have no killing effect on the K562 cell that does not express BCMA, proving that the tumor cell killing effect of CAR-T cells is specific. As can be seen from FIG. 4, at the three effector-to-target ratios, hBCMA-17, hBCMA-19, hBCMA-20, hBCMA-22, and hBCMA-23 of this application are all stronger than the positive control huBCMA-10. At the effector-to-target ratio of 1:1, the killing toxicity against the target cell is greater than 20%. At the effector-to-target ratio of 3:1, the killing toxicity against the target cell is greater than 70%.

**Example 7 IFN-γ secretion after *in vitro* killing of BCMA CAR-T cells**

Referring to Example 6, the target cell killing experiment was carried out in 96-well plates. The effector-target ratio was set at 1:3. The number of effector cells in each 96-well plate was fixed at $2\times10^4$, and the target cell and the effector cell were then added in sequence. Three replicate wells were set at each effector-to-target ratio for each group, and it was required to set three separate effector cell wells for each group. The background factor secretion of the effector cell was then tested.

About 24 hours after the target cell was killed, the cell solution in the 96-well plate was centrifuged and the supernatant was collected from each well (if not tested immediately, the supernatant was required to be stored in a -80 °C refrigerator).

IFN-γ cytokine secretion (R&D, DY285) was tested by the Elisa method as follows:

(1) The IFN-γ capture antibody in the kit was added into a 96-well plate in advance and the plate was coated overnight at room temperature.

(2) The coated 96-well plate was washed with a buffer, a blocking solution was added for blocking at room temperature for 1 hour, and then the 96-well plate was washed with a buffer and set aside for later use.

(3) According to the instructions, the standard product in the kit was gradient diluted, and in the meanwhile the collected supernatant to be tested was diluted by 5-30 folds. The diluted standard product and the sample to be tested were then added into the 96-well plate treated in the above steps, and incubation was then carried out at room temperature for 2 hours.

(4) After all the wells were washed with a buffer, the IFN-$\gamma$ test antibody in the kit was added, and incubation was then carried out at room temperature for 2 hours.

(5) After all the wells were washed with a buffer, Streptavidin-HRP in the kit was added and incubation was then carried out at room temperature in the dark for 20 minutes.

(6) After all the wells were washed with a buffer, a substrate solution TMB (for its formula, please refer to R&D, DY285) was added and incubation was then carried out at room temperature in the dark for 20 minutes.

(7) A stop buffer (for its formula, please refer to R&D, DY285) was added, and the value corresponding to OD450 was read on the ELIASA.

For the working concentrations of various reagents, including IFN-$\gamma$ standard product, capture antibody, test antibody, Streptavidin-HRP, please refer to the instructions (R&D, DY285) for details. For the formulas of other reagents, including the buffer, the blocking solution, the diluent, the substrate solution, and the stop buffer, please refer to R&D, DY285 for details.

Finally, the standard curves and the corresponding fitting formula were obtained according to the four-parameter fitting method. From the fitting formula and the dilution factor of the sample to be tested, the concentration of IFN-$\gamma$ in the sample to be tested was calculated.

As shown in FIG. 5, without stimulation of the MM1s cell, CAR-T cells of all groups did not secrete IFN-$\gamma$. Under the stimulation of the MM1s cell, compared with the positive control huBCMA-10 (Patent Application No. US 2017/0226216 A1) selected in this application, the hBCMA-17, hBCMA-19, hBCMA-20, hBCMA-22, and hBCMA-23 of this patent application can secrete more IFN-$\gamma$ when killing the target cell, with the secretion amount of 1500 pg/mL or above, even up to 2000 pg/mL.

**Example 8 Targeted proliferation ability of BCMA CAR-T cells**

Since the survival rate of the MM1s cell after $\gamma$-ray irradiation is extremely low, untreated MM1s cell was selected as a target cell for testing the targeted proliferation ability of CAR-T cells in this example.

Generally, the testing is carried out after the CAR-T cell is cultured for about 9-12 days. The CAR positive rate of each group needs to be detected in advance, and then blank effector T cell is used to adjust the CAR positive rates of all the groups to be consistent (the culture medium is X-VIVO15 medium without any additives).

A CAR-T cell and the MM1s cell are subjected to the first round of targeted stimulation at an effector-to-target ratio of 1:1. After co-incubation for 4-5 days, all cells are collected, sampled and counted. The second round of targeted stimulation is then carried out at a ratio of 0.5: 1 (total CAR-T cell: new MM1s cell), and after another 4-5 days of co-incubation, the third round of targeted stimulation, where the ratio of the two cells is the same as that in the second round of targeted stimulation). During the three rounds of targeted stimulation, an appropriate amount of X-VIVO15 culture medium is added everyday or every two days according to the cell growth condition.

As shown in FIG. 6, after three rounds of targeted stimulation of the BCMACAR-T cells, the targeted expansion folds of the hBCMA-17, hBCMA-19, hBCMA-20, hBCMA-22, and hBCMA-23 of this application are all high.

**Example 9 *In vitro* targeted killing activity of BCMA CAR-T cells based on the L6-Li CAR structure and IFN$\gamma$ secretion after killing**

As described in Example 2, the nucleic acid sequences of scFv fragments of hBCMA-22 and hu-BCMA-10 were respectively constructed into a CAR lentivirus vector containing L6-Li to obtain the lentivirus vectors of hBCMA-22-L6-Li and hu-BCMA-10-L6-Li. After lentivirus packaging and virus titer determination, and in vitro expansion of CAR-T cells, the in vitro killing activity of CAR-T cells and IFN-$\gamma$ secretion after killing were tested during the period of D8 to D12. For specific operation methods, please refer to Examples 3-7.

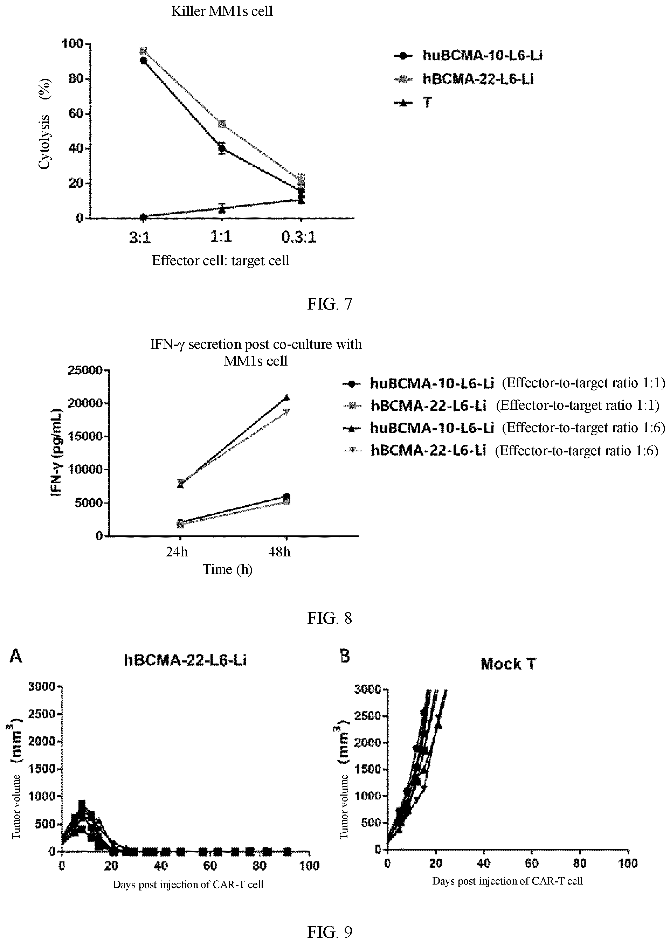

As shown in FIG. 7, the specific killing abilities of CAR-T cells based on hBCMA-22-L6-Li and hu-BCMA-10-L6-Li against MM1s cell at three effector-to-target ratios are basically the same. At the effector-target ratio of 3:1, the killing toxicity of CAR-T cells against the target cell is about 90%. At the effector-target ratio of 1:1, the killing toxicity of CAR-T cells is about 40%. As shown in FIG. 8, the IFN-$\gamma$ secretion abilities of these two groups of CAR-T cells after co-culture with the MM1s cell are basically consistent. At the effector-to-target ratio of 1:1, CAR-T cells and the target cell can stimulate the secretion of IFN-$\gamma$ to approximately 5,000 pg/mL after co-culture for 48 hours. At the effector-to-target ratio of 1:6, CAR-T cells and the target cell can stimulate the secretion of IFN-$\gamma$ to approximately 17500pg/mL after co-culture for 48 hours.

**Example 10 Tumor inhibition and decreasing ability of BCMA CAR-T cells based on the hBCMA-22-L6-Li CAR structure in tumor-bearing mice and their metabolism in mice**

6-week-old mice were prepared and each of the mice was inoculated with $1\times10^7$ MM1s cells subcutaneously. 14 days later, the volumes of the subcutaneous tumors in the mice were about within a range of 150 mm$^3$ to 250 mm$^3$. The mice are divided into two groups (eight mice in each group) and inoculated with freshly prepared CAR-T cells ($2\times10^6$ CAR$^+$/mouse) in the hBCMA-22-L6-Li group and negative control T cells (the number of cells inoculated per mouse in the T cell group was consistent with the total number of cells corresponding to $2\times10^6$ CAR$^+$ in the hBCMA-22-L6-Li group), and defined as D0 after CAR-T injection.

After inoculation with CAR-T cells, twice a week, the tumor size was measured with a vernier caliper, the body weight of the mice was weighed, and the hair color, excrement, food and water intake, body movement, and death of mice were observed. On D7, D14, and D21 after CAR-T injection, blood was collected from the tail vein of 5 mice in each group to detect cytokine secretion (IFN-$\gamma$ and IL-2 cytokines were detected with Th1/2/17 CBA) and the proportion of human T cells (hCD3 and hCD8). The mice were sacrificed when their tumor volumes were greater than 3000 mm$^3$, and all other mice were sacrificed on D90 to end the experiment.

10.1 Detection of hCD3 and hCD8 molecular markers in peripheral blood of mice by flow cytometry

(1) 500 ul of PBS was added into a 10 mL human EDTA.K2 blood collection tube (purchased from Jiangsu Kangjie Medical Devices) and well mixed, and 20 ul was then taken from the blood collection tube and placed into a corresponding 1.5 ml EP tube;

(2) 30 $\mu$l of blood was collected from the tail vein of the mice, and then there was 50 $\mu$l of solution in total;

(3) The solution was centrifuged at 3000rpm for 10 minutes, and 15 $\mu$l of the supernatant was taken out for testing CBA (if not tested immediately, it must be stored in a -80°C refrigerator);

(4) 40 ul of PBS was added to the remaining 35 $\mu$l of solution, and the solution was well mixed again and then transferred to a flow cytometer tube;

(5) APC-Anti-Human-CD3 antibody (BD, 555335) and Percp-cy5.5-Anti-Human-CD8 antibody (BD, 341051) were added;

(6) The solution was well mixed with a vortexer at a low speed and then incubated in a 4 °C refrigerator for 30 minutes;

(7) 2 ml of red blood cell lysis buffer (BD, 34902) was directly added without washing, and the solution was well mixed with a vortexer at a low speed, and then incubated at room temperature in the dark for 10 minutes;

(8) The solution was centrifuged at 500 g for 4 minutes and the centrifuge tube was then placed upside down to discard the supernatant;

(9) 2 ml of the NBS solution in Example 4 was added and the mixed solution was then well mixed with a vortexer at a low speed;

(10) The solution was centrifuged at 500 g for 4 minutes; and

(11) The cell pellet was resuspended in 200 ul of NBS solution and cell resuspension was then well mixed for flow cytometry.

10.2 Method for detecting cytokines (IFN-$\gamma$ and IL-2) with TH1/2/17 CBA (BD, 560484)

(1) Based on the number of samples, the number of beads required was calculated;

(2) Each captured magnetic bead suspension tube (A1-A7) was vortexed vigorously for at least 1 minute;

(3) A 1.5 ml EP tube was used and denoted as TH1/2/17 beads;

(4) The required number of A1-A7 beads were placed in the EP tube;

(5) The mixed beads suspension was vortexed for 1 minute and well mixed;

(6) The suspension was centrifuged at 300 g for 5 minutes;

(7) The supernatant was removed and the TH1/2/17 beads were resuspended in an equal volume of Serum Enhancement Buffer (included in the kit);

(8) After vortexing for 1 minute, the cells were incubated at room temperature in the dark for 30 minutes;

(9) 50 $\mu$l of plasma samples of mice were added into a new EP tube, and then 50 ul of TH1/2/17 beads and 50 $\mu$l

of PE-detection Regent (included in the kit) were added in sequence,
(10) After vortexing for 30s, the cells were incubated at room temperature in the dark for 3 hours;
(11) 500 ul of PBS was added and the solution was well vortexed and then centrifuged at 300 g for 5 minutes; and
(12) The supernatant was discarded, resuspension was carried out in 200 μl of PBS, and the solution was well vortexed and then transferred to a flow cytometer tube for flow cytometer.

As shown in FIGS. 9A and 9B, after the CAR-T cells of the hBCMA-22-L6-Li group were injected into tumor-bearing mice, the tumor volume continued to increase and reached the maximum at D8, which was close to the average tumor volume of the Mock T group at this time. In about two weeks, the tumors of the hBCMA-22-L6-Li group began to decrease, and the tumors completely disappeared in about four weeks. In the third week, the tumor volume of the Mock T group had increased to 3000 mm$^3$ or larger.

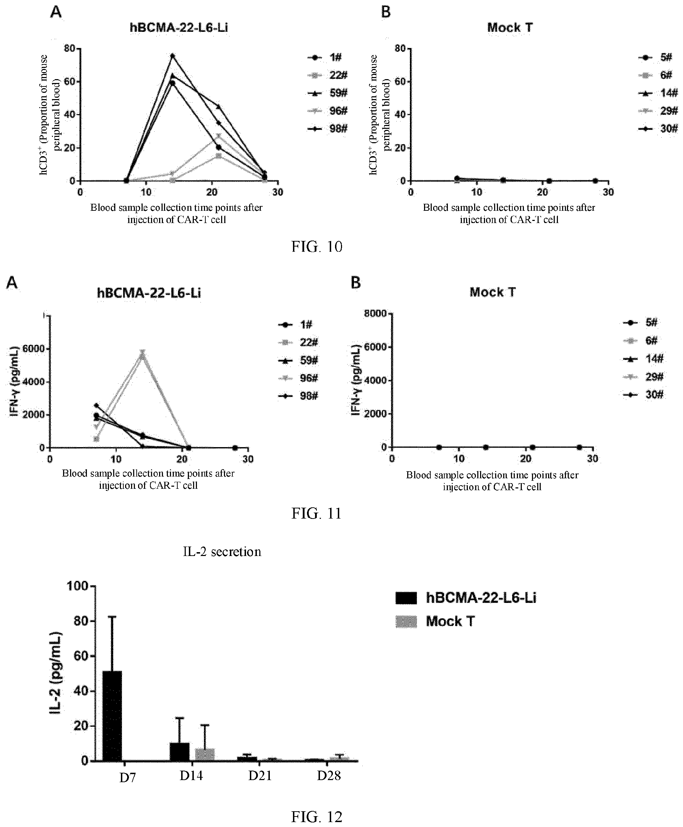

FIGS. 10A and 10B show the proliferation and metabolism of CAR-T cells of the hBCMA-22-L6-Li group in tumor-bearing mice (taking hCD3$^+$ cells as an example, the changing trend of the proportion of hCD8$^+$ cells is basically consistent with that of hCD3$^+$, and no data is shown). The starting point and peak point of rapid expansion of CAR-T cells in different mice are not the same (the peak point of hCD3 may appear between D14 and D21). On the average, the average hCD3$^+$T cell content is about 40% on D14 of CAR-T injection and then gradually decreases. On D28, the average hCD3$^+$T cell content drops to 5% or below. No T cell proliferation in the Mock T group was detected at four detection time points.

While inhibiting and decreasing tumors, a large amount of IFN-γ and a small amount of IL-2 cytokines were detected in the peripheral blood of mice (the secretion of IL-4, IL-6, IL-10, TNF-α, and IL17A cytokines was not detected and no data is shown). As shown in FIG. 11A, the starting point and peak point of IFN-γ cytokine secretion in different mice are also different, and with reference to FIG. 10A, it can be seen that the peaks of IFN-γ cytokine secretion are all earlier than the proliferation peak of CAR-T cell, and the IFN-γ cytokine content drops to 3pg/ml or below on D21. IFN-γ secretion in the Mock T group is not detected at four detection time points.

The secretion level of IL-2 cytokines is significantly lower than that of IFN-γ (FIG. 12) and reaches a peak (50 pg/ml on average) on D7 of CAR-T injection, and then declines rapidly.

The aforementioned details are provided by way of explanations and examples and are not intended to limit the scope of the appended claims. At present, the various changes in the embodiments stated in this application are obvious to those of ordinary skills in the art and fall within the scope of the appended claims and their equivalent solutions.

## Claims

1. A chimeric antigen receptor (CAR), comprising a BCMA binding domain, said BCMA binding domain comprising an antibody or an antigen-binding fragment of the antibody that specifically binds to BCMA, wherein said antibody comprises a heavy-chain complementarity determining region 3 (HCDR3), and said HCDR3 comprises an amino acid sequence as set forth in SEQ ID NO: 25.

2. The CAR of claim 1, wherein said antibody comprises a heavy-chain complementarity determining region 2 (HCDR2), and said HCDR2 comprises an amino acid sequence as set forth in SEQ ID NO: 41.

3. The CAR of claim 2, wherein said HCDR2 comprises an amino acid sequence as set forth in any one of SEQ ID NOs: 22-24.

4. The CAR of any one of claims 1-3, wherein said antibody comprises a heavy-chain complementarity determining region 1 (HCDR1), and said HCDR1 comprises an amino acid sequence as set forth in SEQ ID NO: 21.

5. The CAR of any one of claims 1-4, wherein said antibody comprises an HCDR1, an HCDR2, and an HCDR3, wherein said HCDR1 comprises an amino acid sequence as set forth in SEQ ID NO: 21, said HCDR2 comprises an amino acid sequence as set forth in SEQ ID NO: 41, and said HCDR3 comprises an amino acid sequence as set forth in SEQ ID NO: 25.

6. The CAR of any one of claims 1-5, wherein said antibody comprises an HCDR1, an HCDR2, and an HCDR3, wherein said HCDR1 comprises an amino acid sequence as set forth in SEQ ID NO: 21, said HCDR2 comprises an amino acid sequence as set forth in any one of SEQ ID NOs: 22-24, and said HCDR3 comprises an amino acid sequence as set forth in SEQ ID NO: 25.

7. The CAR of any one of claims 1-6, wherein said antibody comprises any set of HCDR1, HCDR2, and HCDR3, selected from the groups consisting of:

> (1) HCDR1: SEQ ID NO: 21, HCDR2: SEQ ID NO: 22, and HCDR3: SEQ ID NO: 25;
> (2) HCDR1: SEQ ID NO: 21, HCDR2: SEQ ID NO: 23, and HCDR3: SEQ ID NO: 25; and
> (3) HCDR1: SEQ ID NO: 21, HCDR2: SEQ ID NO: 24, and HCDR3: SEQ ID NO: 25.

8. The CAR of any one of claims 1-7, wherein said antibody comprises a heavy chain variable region (VH), said VH comprises a framework region H-FR1, a C terminal of said H-FR1 is directly or indirectly linked to an N terminal of said HCDR1, and said H-FR1 comprises an amino acid sequence as set forth in SEQ ID NO: 42.

9. The CAR of claim 8, wherein said H-FR1 comprises an amino acid sequence as set forth in any one of SEQ ID NOs: 26-29.

10. The CAR of any one of claims 8-9, wherein said VH comprises a framework region H-FR2, and said H-FR2 is located between said HCDR1 and said HCDR2 and comprises an amino acid sequence as set forth in SEQ ID NO: 43.

11. The CAR of claim 10, wherein said H-FR2 comprises an amino acid sequence as set forth in any one of SEQ ID NOs: 30-32.

12. The CAR of any one of claims 8-11, wherein said VH comprises a framework region H-FR3, and said H-FR3 is located between said HCDR2 and said HCDR3 and comprises an amino acid sequence as set forth in SEQ ID NO: 44.

13. The CAR of claim 12, wherein said H-FR3 comprises an amino acid sequence as set forth in any one of SEQ ID NOs: 33-36.

14. The CAR of any one of claims 8-13, wherein said VH comprises a framework region H-FR4, an N terminal of said H-FR4 is linked to a C terminal of said HCDR3, and said H-FR4 comprises an amino acid sequence as set forth in SEQ ID NO: 37.

15. The CAR of any one of claims 1-14, wherein said antibody comprises an H-FR1, an H-FR2, an H-FR3, and an H-FR4, wherein said H-FR1 comprises an amino acid sequence as set forth in SEQ ID NO: 42, said H-FR2 comprises an amino acid sequence as set forth in SEQ ID NO: 43, said H-FR3 comprises an amino acid sequence as set forth in SEQ ID NO: 44, and said H-FR4 comprises an amino acid sequence as set forth in SEQ ID NO: 37.

16. The CAR of any one of claims 1-15, wherein said H-FR1 comprises an amino acid sequence as set forth in any one of SEQ ID NOs: 26-29, said H-FR2 comprises an amino acid sequence as set forth in any one of SEQ ID NOs: 30-32, said H-FR3 comprises an amino acid sequence as set forth in any one of SEQ ID NOs: 33-36, and said H-FR4 comprises an amino acid sequence as set forth in SEQ ID NO: 37.

17. The CAR of any one of claims 1-16, wherein said H-FR1, H-FR2, H-FR3, and H-FR4 comprises any set of amino acid sequence selected from the groups consisting of:

> (1) H-FR1: SEQ ID NO: 26, H-FR2: SEQ ID NO: 30, H-FR3: SEQ ID NO: 33, and H-FR4: SEQ ID NO: 37;
> (2) H-FR1: SEQ ID NO: 27, H-FR2: SEQ ID NO: 31, H-FR3: SEQ ID NO: 34, and H-FR4: SEQ ID NO: 37;
> (3) H-FR1: SEQ ID NO: 28, H-FR2: SEQ ID NO: 32, H-FR3: SEQ ID NO: 35, and H-FR4: SEQ ID NO: 37; and
> (4) H-FR1: SEQ ID NO: 29, H-FR2: SEQ ID NO: 30, H-FR3: SEQ ID NO: 36, and H-FR4: SEQ ID NO: 37.

18. The CAR of any one of claims 1-17, wherein said antibody comprises a heavy chain variable region (VH), and said VH comprises an amino acid sequence as set forth in SEQ ID NO: 46.

19. The CAR of claim 18, wherein said VH comprises an amino acid sequence as set forth in any one of SEQ ID NOs: 5-9.

20. The CAR of any one of claims 1-19, wherein said antibody comprises a light-chain complementarity determining region 3 (LCDR3), and said LCDR3 comprises an amino acid sequence as set forth in SEQ ID NO: 40.

21. The CAR of claim 20, wherein said LCDR3 comprises an amino acid sequence as set forth in any one of SEQ ID NOs: 15-16.

22. The CAR of any one of claims 1-21, wherein said antibody comprises a light-chain complementarity determining region 2 (LCDR2), and said LCDR2 comprises an amino acid sequence as set forth in SEQ ID NO: 39.

23. The CAR of claim 22, wherein said LCDR2 comprises an amino acid sequence as set forth in any one of SEQ ID NOs: 13-14.

24. The CAR of any one of claims 1-23, wherein said antibody comprises a light-chain complementarity determining region 1 (LCDR1), and said LCDR1 comprises an amino acid sequence as set forth in SEQ ID NO: 38.

25. The CAR of claim 24, wherein said LCDR1 comprises an amino acid sequence as set forth in any one of SEQ ID NOs: 10-12.

26. The CAR of any one of claims 1-25, wherein said antibody comprises an LCDR1, an LCDR2, and an LCDR3, wherein said LCDR1 comprises an amino acid sequence as set forth in SEQ ID NO: 38, said LCDR2 comprises an amino acid sequence as set forth in SEQ ID NO: 39, and said LCDR3 comprises an amino acid sequence as set forth in SEQ ID NO: 40.

27. The CAR of any one of claims 1-26, wherein said antibody comprises an LCDR1, an LCDR2, and an LCDR3, wherein said LCDR1 comprises an amino acid sequence as set forth in any one of SEQ ID NOs: 10-12, said LCDR2 comprises an amino acid sequence as set forth in any one of SEQ ID NO: 13-14, and said LCDR3 comprises an amino acid sequence as set forth in any one of SEQ ID NO: 15-16.

28. The CAR of any one of claims 1-27, wherein said antibody comprises any set of LCDR1, LCDR2, and LCDR3, selected from the groups consisting of:

    (1) LCDR1: SEQ ID NO: 10, LCDR2: SEQ ID NO: 13, and LCDR3: SEQ ID NO: 15;
    (2) LCDR1: SEQ ID NO: 11, LCDR2: SEQ ID NO: 13, and LCDR3: SEQ ID NO: 16;
    (3) LCDR1: SEQ ID NO: 12, LCDR2: SEQ ID NO: 13, and LCDR3: SEQ ID NO: 16; and
    (4) LCDR1: SEQ ID NO: 12, LCDR2: SEQ ID NO: 14, and LCDR3: SEQ ID NO: 16.

29. The CAR of any one of claims 1-28, wherein said antibody comprises a light chain variable region (VL), said VL comprises a framework region L-FR1, a C terminal of said L-FR1 is directly or indirectly linked to an N terminal of said LCDR1, and said L-FR1 comprises an amino acid sequence as set forth in SEQ ID NO: 17.

30. The CAR of claim 29, wherein said VL comprises a framework region L-FR2, and said L-FR2 is located between said LCDR1 and said LCDR2 and comprises an amino acid sequence as set forth in SEQ ID NO: 18.

31. The CAR of any one of claims 29-30, wherein said VL comprises a framework region L-FR3, and said L-FR3 is located between said LCDR2 and said LCDR3 and comprises an amino acid sequence as set forth in SEQ ID NO: 19.

32. The CAR of any one of claims 29-31, wherein said VL comprises a framework region L-FR4, an N terminal of said L-FR4 is linked to a C terminal of said LCDR3, and said L-FR4 comprises an amino acid sequence as set forth in SEQ ID NO: 20.

33. The CAR of any one of claims 1-32, wherein said antibody comprises an L-FR1, an L-FR2, an L-FR3, and an L-FR4, wherein said L-FR1 comprises an amino acid sequence as set forth in SEQ ID NO: 17, said L-FR2 comprises an amino acid sequence as set forth in SEQ ID NO: 18, said L-FR3 comprises an amino acid sequence as set forth in SEQ ID NO: 19, and said L-FR4 comprises an amino acid sequence as set forth in SEQ ID NO: 20.

34. The CAR of any one of claims 1-33, wherein said antibody comprises a light chain variable region (VL), and said VL comprises an amino acid sequence as set forth in SEQ ID NO: 45.

35. The CAR of claim 34, wherein said VL comprises an amino acid sequence as set forth in any one of SEQ ID NOs: 1-4.

36. The CAR of any one of claims 1-35, wherein said antibody comprises a VH and a VL, said VH comprises an amino acid sequence as set forth in SEQ ID NO: 46, and said VL comprises an amino acid sequence as set forth in SEQ ID NO: 45.

37. The CAR of any one of claims 1-36, wherein said antibody comprises a VH and a VL, said VH comprises an amino acid sequence as set forth in any one of SEQ ID NOs: 5-9, and said VL comprises an amino acid sequence as set forth in any one of SEQ ID NOs: 1-4.

38. The CAR of any one of claims 1-37, wherein said antibody comprises a VH and a VL, and said VH and VL comprises any set of amino acid sequence, selected from the groups consisting of:

    (1) VH: SEQ ID NO: 5 and VL: SEQ ID NO: 1;
    (2) VH: SEQ ID NO: 6 and VL: SEQ ID NO: 2;
    (3) VH: SEQ ID NO: 7 and VL: SEQ ID NO: 2;
    (4) VH: SEQ ID NO: 8 and VL: SEQ ID NO: 2;
    (5) VH: SEQ ID NO: 9 and VL: SEQ ID NO: 3;
    (6) VH: SEQ ID NO: 8 and VL: SEQ ID NO: 3;
    (7) VH: SEQ ID NO: 9 and VL: SEQ ID NO: 4; and
    (8) VH: SEQ ID NO: 8 and VL: SEQ ID NO: 4.

39. The CAR of any one of claims 1-38, wherein said BCMA binding domain comprises a scFv, and said scFv comprises the VH and the VL of said antibody that specifically binds to BCMA.

40. The CAR of claim 39, wherein the C terminal of said VH and the N terminal of said VL are linked directly or indirectly.

41. The CAR of any one of claims 39-40, wherein the C terminal of said VL and the N terminal of said VL are linked directly or indirectly.

42. The CAR of any one of claims 39-41, wherein said scFv comprises a linker peptide between said VH and said VL, and said linker peptide comprises an amino acid sequence as set forth in SEQ ID NO: 58.

43. The CAR of any one of claims 39-42, wherein said scFv comprises an amino acid sequence as set forth in any one of SEQ ID NOs: 49-56.

44. The CAR of any one of claims 1-43 comprising a transmembrane domain, wherein said transmembrane domain comprises transmembrane domains derived from proteins selected from the group consisting of: CD28, CD3e, CD45, CD4, CD5, CD8, CD9, CD16, CD22, CD33, CD37, CD64, CD80, CD86, CD134, CD137, and CD154.

45. The CAR of claim 44, wherein said transmembrane domain is derived from CD8, and said transmembrane domain comprises an amino acid sequence as set forth in SEQ ID NO: 61.

46. The CAR of any one of claims 1-45 comprising a costimulatory domain, said costimulatory domain comprises costimulatory domains derived from proteins selected from the group consisting of: CD137, CD28, 4-1BB, OX-40, and ICOS.

47. The CAR of claim 46, wherein said costimulatory domain is derived from CD137, and said costimulatory domain comprises an amino acid sequence as set forth in SEQ ID NO: 62.

48. The CAR of any one of claims 1-47, comprising an intracellular signaling domain, wherein said intracellular signaling domain comprises an intracellular signaling domain derived from CD3ζ.

49. The CAR of claim 48, wherein said intracellular signaling domain comprises an amino acid sequence as set forth in SEQ ID NO: 63.

50. The CAR of any one of claims 1-49, comprising a hinge region, wherein said hinge region comprises a hinge region derived from CD8.

51. The CAR of claim 50, wherein said hinge region is derived from CD8 and comprises an amino acid sequence as set forth in SEQ ID NO: 60.

52. The CAR of any one of claims 1-51, comprising a signal peptide.

53. The CAR of claim 52, wherein said signal peptide comprises an amino acid sequence as set forth in SEQ ID NO: 59.

54. The CAR of any one of claims 1-53, comprising L6-Li, wherein said L6-Li is located at a C terminal of said intracellular signaling domain.

55. The CAR of claim 54, wherein said L6-Li comprises an amino acid sequence as set forth in SEQ ID NO: 107.

56. The CAR of any one of claims 1-55, comprising an amino acid sequence as set forth in any one of SEQ ID NOs: 64-71 and 74.

57. The CAR of any one of claims 1-56, comprising a nucleotide sequence as set forth in any one of SEQ ID NOs: 95-102 and 105.

58. An isolated nucleic acid molecule, comprising a nucleotide sequence encoding the CAR of any one of claims 1-57.

59. The isolated nucleic acid molecule of claim 58, further comprising a promoter, said promotor is located at a 5' terminal of the nucleotide sequence encoding said CAR.

60. The isolated nucleic acid molecule of any one of claims 58-59, wherein said promoter is a constitutive promoter.

61. The isolated nucleic acid molecule of any one of claims 58-60, wherein said promoter is an EF1$\alpha$ promoter.

62. A nucleic acid molecule encoding a CAR, said nucleic acid molecule comprising a nucleotide sequence as set forth in any one of SEQ ID NOs: 95-102 and 105.

63. A vector, comprising the nucleic acid molecule of any one of claims 58-62.

64. The vector of claim 63, wherein said vector is selected from the group consisting of plasmids, retroviral vectors, and lentivirus vectors.

65. An immune effector cell, comprising the CAR of any one of claims 1-57, the nucleic acid molecule of any one of claims 58-62, and/or the vector of any one of claims 63-64.

66. The cell of claim 65, wherein said immune effector cell is selected from T lymphocytes.

67. A method for preparing an immune effector cell, comprising: introducing the vector of any one of claims 63-64 into said immune effector cell.

68. A pharmaceutical composition, comprising the CAR of any one of claims 1-57, the nucleic acid molecule of any one of claims 58-62, the vector of any one of claims 63-64, and/or the immune effector cell of any one of claims 65-66, and a pharmaceutically acceptable carrier.

69. A use of the CAR of any one of claims 1-57, the nucleic acid molecule of any one of claims 58-62, the vector of any one of claims 63-64, the immune effector cell of any one of claims 65-66, and/or the pharmaceutical composition of claim 68 in the manufacture of a medicament, wherein said medicament is used for treating a disease and/or disorder associated with BCMA expression.

70. The use of claim 69, wherein said disease or disorder associated with BCMA expression is a cancer or a malignant tumor.

71. The use of any one of claims 69-70, wherein said disease or disorder associated with BCMA expression is a BCMA-positive tumor.

72. The use of any one of claims 69-71, wherein said disease or disorder associated with BCMA expression is a solid tumor and/or a non-solid tumor.

73. The use of any one of claims 69-72, wherein said disease or disorder associated with BCMA expression is myeloma.

74. The use of any one of claims 69-73, wherein said disease or disorder associated with BCMA expression is multiple myeloma.

75. A method for treating a disease or disorder associated with BCMA expression, comprising: administering the CAR of any one of claims 1-57, the nucleic acid molecule of any one of claims 58-62, the vector of any one of claims 63-64, the immune effector cell of any one of claims 65-66, and/or the pharmaceutical composition of claim 68 to a subject in need.

76. The method of claim 75, wherein said disease or disorder associated with BCMA expression is a cancer or a malignant tumor.

77. The method of any one of claims 75-76, wherein said disease or disorder associated with BCMA expression is a BCMA-positive tumor.

78. The method of any one of claims 75-77, wherein said disease or disorder associated with BCMA expression is a solid tumor and/or a non-solid tumor.

79. The method of any one of claims 75-78, wherein said disease or disorder associated with BCMA expression is myeloma.

80. The method of any one of claims 75-79, wherein said disease or disorder associated with BCMA expression is multiple myeloma.

81. The CAR of any one of claims 1-57, the nucleic acid molecule of any one of claims 58-62, the vector of any one of claims 63-64, the immune effector cell of any one of claims 65-66, and/or the pharmaceutical composition of claim 68, for use in treating a disease and/or disorder associated with BCMA expression.

82. The CAR, the nucleic acid molecule, the vector, the immune effector cell, and/or the pharmaceutical composition of claim 81, wherein said disease or disorder associated with BCMA expression is a cancer or a malignant tumor.

83. The CAR, the nucleic acid molecule, the vector, the immune effector cell, and/or the pharmaceutical composition of any one of claims 81-82, wherein said disease or disorder associated with BCMA expression is a BCMA-positive tumor.

84. The CAR, the nucleic acid molecule, the vector, the immune effector cell, and/or the pharmaceutical composition of any one of claims 81-83, wherein said disease or disorder associated with BCMA expression is a solid tumor and/or a non-solid tumor.

85. The CAR, the nucleic acid molecule, the vector, the immune effector cell, and/or the pharmaceutical composition of any one of claims 81-84, wherein said disease or disorder associated with BCMA expression is myeloma.

86. The CAR, the nucleic acid molecule, the vector, the immune effector cell, and/or the pharmaceutical composition of any one of claims 81-85, wherein said disease or disorder associated with BCMA expression is multiple myeloma.

FIG. 1

FIG. 2

FIG. 3

A

Killer MM1s cell

B

Killer K562 cell

FIG. 4

FIG. 5

FIG. 6

Killer MM1s cell

FIG. 7

IFN-γ secretion post co-culture with MM1s cell

FIG. 8

A

B

FIG. 9

FIG. 10

FIG. 11

IL-2 secretion

FIG. 12

## INTERNATIONAL SEARCH REPORT

| | International application No. |
|---|---|
| | **PCT/CN2022/089287** |

**A. CLASSIFICATION OF SUBJECT MATTER**

A61P 35/00(2006.01)i; C07K 16/28(2006.01)i; A61K 35/17(2015.01)i; C12N 15/13(2006.01)i; C12N 5/10(2006.01)i; C07K 19/00(2006.01)i

According to International Patent Classification (IPC) or to both national classification and IPC

**B. FIELDS SEARCHED**

Minimum documentation searched (classification system followed by classification symbols)

A61P,C07K,A61K,C12N

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, JPTXT, USTXT, EPTXT, 万方, WANFANG, CNKI, PubMed, GenBank, ISI web of knowledge: BCMA, 嵌合抗原受体(CAR), BCMA结合结构, 轻链互补决定区, 重链互补决定区, 免疫效应细胞, 药物组合物, BCMA chimeric antigen receptors, adoptive T cell therapies, CAR cells

**C. DOCUMENTS CONSIDERED TO BE RELEVANT**

| Category* | Citation of document, with indication, where appropriate, of the relevant passages | Relevant to claim No. |
|---|---|---|
| A | CN 109942709 A (GUANGZHOU BIO-GENE TECHNOLOGY CO., LTD.) 28 June 2019 (2019-06-28) <br> claims 1-10, and description, paragraphs 11-17, 37-46, and 142-145, embodiments, the drawings, and sequences 67 and 68 | 1-86 |
| A | CN 111944054 A (CHONGQING PRECISION BIOTECHNOLOGY CO., LTD.) 17 November 2020 (2020-11-17) <br> claims 1-20, and sequences 1-10 | 1-86 |
| A | CN 111918964 A (SHANGHAI ORIGINCELL MEDICAL TECHNOLOGY CO., LTD.) 10 November 2020 (2020-11-10) <br> entire document | 1-86 |
| A | CN 109134665 A (SHENZHEN PREGENE BIOPHARMACEUTICAL CO., LTD.) 04 January 2019 (2019-01-04) <br> entire document | 1-86 |
| A | CN 110662771 A (NANJING IASO BIOTHERAPEUTICS CO., LTD. et al.) 07 January 2020 (2020-01-07) <br> entire document | 1-86 |

☑ Further documents are listed in the continuation of Box C.    ☑ See patent family annex.

| | |
|---|---|
| \*   Special categories of cited documents: <br> "A"   document defining the general state of the art which is not considered to be of particular relevance <br> "E"   earlier application or patent but published on or after the international filing date <br> "L"   document which may throw doubts on priority claim(s) or which is cited to establish the publication date of another citation or other special reason (as specified) <br> "O"   document referring to an oral disclosure, use, exhibition or other means <br> "P"   document published prior to the international filing date but later than the priority date claimed | "T"   later document published after the international filing date or priority date and not in conflict with the application but cited to understand the principle or theory underlying the invention <br> "X"   document of particular relevance; the claimed invention cannot be considered novel or cannot be considered to involve an inventive step when the document is taken alone <br> "Y"   document of particular relevance; the claimed invention cannot be considered to involve an inventive step when the document is combined with one or more other such documents, such combination being obvious to a person skilled in the art <br> "&"   document member of the same patent family |

| Date of the actual completion of the international search | Date of mailing of the international search report |
|---|---|
| **24 May 2022** | **27 July 2022** |

| Name and mailing address of the ISA/CN | Authorized officer |
|---|---|
| **China National Intellectual Property Administration (ISA/CN)** <br> **No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.

**PCT/CN2022/089287**

**C.     DOCUMENTS CONSIDERED TO BE RELEVANT**

| Category* | Citation of document, with indication, where appropriate, of the relevant passages | Relevant to claim No. |
|---|---|---|
| A | WO 2020097403 A1 (JUNO THERAPEUTICS, INC.) 14 May 2020 (2020-05-14) entire document | 1-86 |
| A | WO 2016014789 A2 (BLUEBIRD BIO, INC.) 28 January 2016 (2016-01-28) entire document | 1-86 |

Form PCT/ISA/210 (second sheet) (January 2015)

**INTERNATIONAL SEARCH REPORT**

International application No.

**PCT/CN2022/089287**

| Box No. I | Nucleotide and/or amino acid sequence(s) (Continuation of item 1.c of the first sheet) |

1. With regard to any nucleotide and/or amino acid sequence disclosed in the international application, the international search was carried out on the basis of a sequence listing:

    a. ☑ forming part of the international application as filed:

        ☑ in the form of an Annex C/ST.25 text file.

        ☐ on paper or in the form of an image file.

    b. ☐ furnished together with the international application under PCT Rule 13*ter*.1(a) for the purposes of international search only in the form of an Annex C/ST.25 text file.

    c. ☐ furnished subsequent to the international filing date for the purposes of international search only:

        ☐ in the form of an Annex C/ST.25 text file (Rule 13*ter*.1(a)).

        ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**

| International application No. |
| --- |
| **PCT/CN2022/089287** |

| Box No. II    Observations where certain claims were found unsearchable (Continuation of item 2 of first sheet) |
| --- |

This international search report has not been established in respect of certain claims under Article 17(2)(a) for the following reasons:

1. [✓] Claims Nos.: **75-80**
   because they relate to subject matter not required to be searched by this Authority, namely:

   [1]   Claims 75-80 relate to a method for treatment or diagnosis of a living human or animal body, falling
   within the cases listed in PCT Rule 39.1(iv) for which no international search is required. In the preset
   report, a search was conducted on the basis of "a use in the preparation of a drug for treating diseases
   or illnesses associated with BCMA expression".

2. [ ] Claims Nos.:
   because they relate to parts of the international application that do not comply with the prescribed requirements to such an
   extent that no meaningful international search can be carried out, specifically:

3. [ ] Claims Nos.:
   because they are dependent claims and are not drafted in accordance with the second and third sentences of Rule 6.4(a).

Form PCT/ISA/210 (continuation of first sheet) (January 2015)

| INTERNATIONAL SEARCH REPORT | | | | | | |
|---|---|---|---|---|---|---|
| Information on patent family members | | | | International application No. PCT/CN2022/089287 | | |

| Patent document cited in search report | | | Publication date (day/month/year) | Patent family member(s) | | | Publication date (day/month/year) |
|---|---|---|---|---|---|---|---|
| CN | 109942709 | A | 28 June 2019 | None | | | |
| CN | 111944054 | A | 17 November 2020 | None | | | |
| CN | 111918964 | A | 10 November 2020 | WO | 2019184886 | A1 | 03 October 2019 |
| | | | | JP | 2021518149 | A | 02 August 2021 |
| | | | | US | 2021106620 | A1 | 15 April 2021 |
| | | | | EP | 3778876 | A1 | 17 February 2021 |
| CN | 109134665 | A | 04 January 2019 | CA | 3109320 | A1 | 27 February 2020 |
| | | | | EP | 3842462 | A1 | 30 June 2021 |
| | | | | JP | 2021535756 | A | 23 December 2021 |
| | | | | BR | 112021003408 | A2 | 18 May 2021 |
| | | | | AU | 2019325036 | A1 | 22 April 2021 |
| | | | | WO | 2020038146 | A1 | 27 February 2020 |
| | | | | SG | 11202101685 X | A | 30 March 2021 |
| | | | | KR | 20200023165 | A | 04 March 2020 |
| | | | | EA | 202190607 | A1 | 28 May 2021 |
| CN | 110662771 | A | 07 January 2020 | US | 2020246381 | A1 | 06 August 2020 |
| | | | | TW | 201934579 | A | 01 September 2019 |
| | | | | WO | 2019149250 | A1 | 08 August 2019 |
| | | | | EP | 3674327 | A1 | 01 July 2020 |
| WO | 2020097403 | A1 | 14 May 2020 | AU | 2019377854 | A1 | 27 May 2021 |
| | | | | JP | 2022512971 | A | 07 February 2022 |
| | | | | CA | 3117978 | A1 | 14 May 2020 |
| | | | | US | 2022008477 | A1 | 13 January 2022 |
| | | | | KR | 20210111247 | A | 10 September 2021 |
| | | | | EP | 3876958 | A1 | 15 September 2021 |
| | | | | IL | 282886 | D0 | 30 June 2021 |
| | | | | CN | 113573719 | A | 29 October 2021 |
| | | | | SG | 11202104411V | A | 28 May 2021 |
| WO | 2016014789 | A2 | 28 January 2016 | ZA | 201700730 | B | 30 June 2021 |
| | | | | US | 2017226216 | A1 | 10 August 2017 |
| | | | | US | 2020079864 | A1 | 12 March 2020 |
| | | | | CA | 2956002 | A1 | 28 January 2016 |
| | | | | MX | 2017001079 | A | 12 September 2017 |
| | | | | JP | 2017522882 | A | 17 August 2017 |
| | | | | RU | 2017106025 | A | 30 August 2018 |
| | | | | KR | 20170036021 | A | 31 March 2017 |
| | | | | IL | 250202 | D0 | 30 March 2017 |
| | | | | SG | 11201700492 S | A | 27 February 2017 |
| | | | | SG | 10201900455 Y | A | 27 February 2019 |
| | | | | BR | 112017001498 | A2 | 20 February 2018 |
| | | | | EP | 3172231 | A2 | 31 May 2017 |
| | | | | ES | 2878449 | T3 | 18 November 2021 |
| | | | | CN | 106795217 | A | 31 May 2017 |
| | | | | AU | 2015292590 | A1 | 16 February 2017 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021057932 A1

- US 20170226216 A1

**Non-patent literature cited in the description**

- **SAMBROOK et al.** Molecular Cloning, A Laboratory Manual. Cold Spring Harbor Laboratory Press, 1989

- **AUSUBE et al.** Current Protocols in Molecular Biology. Greene Publishing and Wiley-Interscience, 1993